# EUROPEAN PATENT SPECIFICATION

(11) **EP 2 289 908 B1**
(45) Date of publication and mention of the grant of the patent: **07.08.2013**
(21) Application number: 10182824.2
(22) Date of filing: 08.07.2004
(51) Int. Cl.: C07K 14/435, A61K 38/17, A61P 3/00

(54) **Use of DG177 secreted protein products for preventing and treating pancreatic diseases and/or obesity and/or metabolic syndrome**
Verwendung von DG177 sekretierter Proteinprodukte zur Prevention und Behandlung von Pankreaserkrankungen und/oder Fettleibigkeit und/oder metabolischem Syndrom
Utilisation de produits des protéines DG177 sécretées pour la prévention et le traitement de maladies du pancreas et/ou de l'obésité et/ou du syndrome métabolique

(30) Priority: 11.07.2003 EP 03015883; 22.07.2003 EP 03016710
(43) Date of publication of application: 02.03.2011
(62) Divisional of application: 04763138.7
(73) Proprietor: Evotec International GmbH, 22419 Hamburg (DE)
(72) Inventor: Onichtchouk, Daria, 37077, Göttingen (DE)
(74) Representative: Weiss, Wolfgang

(56) References cited:
- EP-A1- 0 869 176
- WO-A2-03/072714
- WO-A2-03/102163
- WO-A2-2004/020465
- WO-A2-2005/033134
- SCHNEYER ALAN ET AL: "Follistatin-related protein (FSRP): A new member of the follistatin gene family", MOLECULAR AND CELLULAR ENDOCRINOLOGY, vol. 180, no. 1-2, 30 June 2001 (2001-06-30), pages 33-38, XP009143172, & PROCEEDINGS OF THE INTERNATIONAL WORKSHOP ON INHIBINS, ACTIVINS AND FOLLISTATINS; MELBOURNE, AUSTRALIA; OCTOBER 26-28, 2000 ISSN: 0303-7207
- KAWABATA D ET AL: "Ameliorative effects of follistatin-related protein/TSC-36/FSTL1 on joint inflammation in a mouse model of arthritis", ARTHRITIS & RHEUMATISM, JOHN WILEY & SONS, INC, US, vol. 50, no. 2, 1 February 2004 (2004-02-01), pages 660-668, XP002998883, ISSN: 0004-3591, DOI: DOI:10.1002/ART.20023

## Description

This invention relates to the use of low molecular weight DG177 proteins, to the use of polynucleotides encoding these, in the diagnosis, study, prevention, and treatment of pancreatic diseases selected from diabetes (e.g. diabetes mellitus), obesity and/or metabolic syndrome and to the use in regeneration of tissues such as pancreatic tissues and others.

Many human proteins serve as pharmaceutically active compounds. Several classes of human proteins that serve as such active compounds include hormones, cytokines, cell growth factors, and cell differentiation factors. Most proteins that can be used as a pharmaceutically active compound fall within the family of secreted proteins. Secreted proteins are generally produced within cells at rough endoplasmic reticulum, are then exported to the golgi complex, and then move to secretory vesicles or granules, where they are secreted to the exterior of the cell via exocytosis. Examples for commercially used secreted proteins are human insulin, thrombolytic agents, interferons, interleukins, colony stimulating factors, human growth hormone, transforming growth factor beta, tissue plasminogen activator, erythropoeitin, and various other proteins. Receptors of secreted proteins, which are membrane-bound proteins, also have potential as therapeutic or diagnostic agents. It is, therefore, important for developing new pharmaceutical compounds to identify secreted proteins that can be tested for activity in a variety of animal models. Thus, in light of the pervasive role of secreted proteins in human physiology, a need exists for identifying and characterizing novel functions for human secreted proteins and the genes that encode them. This knowledge will allow one to detect, to treat, and to prevent medical diseases, disorders, and/or conditions by using secreted proteins or the genes that encode them.

The pancreas is an essential organ possessing both an exocrine function involved in the delivery of enzymes into the digestive tract and an endocrine function by which various hormones are secreted into the blood stream. The exocrine function is assured by acinar and centroacinar cells that produce various digestive enzymes and intercalated ducts that transport these enzymes in alkaline solution to the duodenum. The functional unit of the endocrine pancreas is the islet of Langerhans. Islets are scattered throughout the exocrine portion of the pancreas and are composed of four cell types: alpha-, beta-, delta- and PP-cells, reviewed for example in Kim S.K. and Hebrok M., (2001) Genes Dev. 15: 111-127. Beta-cells produce insulin, represent the majority of the endocrine cells and form the core of the islets, while alpha-cells secrete glucagon and are located in the periphery. Delta-cells and PP-cells are less numerous and secrete somatostatin and pancreatic polypeptide, respectively.

Early pancreatic development has been well studied in different species, including chicken, zebrafish, and mice (for an detailed review, see Kim & Hebrock, 2001, supra). The pancreas develops from distinct dorsal and ventral anlagen. Pancreas development requires specification of the pancreas structure along both anterior-posterior and dorsal-ventral axes. A number of transcription factors, which are critical for proper pancreatic development have been identified (see Kim & Hebrok, 2001, supra; Wilson M.E. et al., (2003) Mech Dev. 120: 65-80).

In postnatal/adult humans, the acinar and ductal cells retain a significant proliferative capacity that can ensure cell renewal and growth, whereas the islet cells become mostly mitotically inactive. This is in contrast to rodents where beta-cell replication is an important mechanism in the generation of new beta cells. It has been suggested, that during embryonic development, pancreatic islets of Langerhans originate from differentiating duct cells or other cells with epithelial morphology (Bonner-Weir S. and Sharma A., (2002) J Pathol. 197: 519-526; Gu G. et al., (2003) Mech Dev. 120: 35-43). In adult humans, new beta cells arise in the vicinity of ducts (Butler A.E. et al., (2003) Diabetes 52: 102-110; Bouwens L. and Pipeleers D.G., (1998) Diabetologia 41: 629-633). However, also an intra-islet location or an origin in the bone marrow has been suggested for precursor cells of adult beta cells (Zulewski H. et al., (2001) Diabetes 50: 521-533; Ianus A. et al., (2003) J Clin Invest. 111: 843-850). Pancreatic islet growth is dynamic and responds to changes in insulin demand, such as during pregnancy or during the increase in body mass occuring during childhood. In adults, there is a good correlation between body mass and islet mass (Yoon K.H. et al., (2003) J Clin Endocrinol Metab. 88: 2300-2308).

Pancreatic beta-cells secrete insulin, which is stimulated by high blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus the amount of insulin produced by the pancreatic islet cells is too low, resulting in elevated blood glucose levels (hyperglycemia). In diabetes type 1 beta cells are lost due to autoimmune destruction. In type 2 diabetic patients, liver and muscle cells loose their ability to respond to normal blood insulin levels (insulin resistance). High blood glucose levels (and also high blood lipid levels) lead to an impairment of beta-cell function and to an increase in beta-cell apoptosis. It is interesting to note that the rate of beta-cell neogenesis does not appear to change in type 2 diabetics (Butler et al., 2003, supra), thus causing a reduction in total beta-cell mass over time. Eventually the application of exogenous insulin becomes necessary in type 2 diabetics.

Improving metabolic parameters such as blood sugar and blood lipid levels (e.g. through dietary changes, exercise, medication or combinations thereof) before beta cell mass has fallen below a critical threshold leads to a relatively rapid restoration of beta cell function. However, after such a treatment the pancreatic endocrine function would remain impaired due to the only slightly increased regeneration rate.

In type 1 diabetics, the lifespan of pancreatic islets is dramatically shortened due to autoimmune destruction. Treatments have been devised which modulate the immune system and may be able to stop or strongly reduce islet destruction (Raz I. et al., (2001) Lancet 358: 1749-1753; Chatenoud L. et al., (2003) Nat Rev Immunol. 3: 123-132). However, due to the relatively slow regeneration of human beta cells such treatments could only be fully successful at improving the diabetic condition if they are combined with an agent which can stimulate beta cell regeneration. Thus, both for type 1 and type 2 diabetes (early and late stages) there is a need to find novel agents which stimulate beta cell regeneration.

Diabetes is a very disabling disease, because medications do not control blood sugar levels well enough to prevent swinging between high and low blood sugar levels. Patients with diabetes are at risk for major complications, including diabetic ketoacidosis, end-stage renal disease, diabetic retinopathy and amputation. There are also a host of related conditions, such as metabolic syndrome, obesity, hypertension, heart disease, peripheral vascular disease, and infections, for which persons with diabetes are at substantially increased risk. The treatment of these complications contributes to a considerable degree to the enormous cost which is imposed by diabetes on health care systems world wide.

Obesity is one of the most prevalent metabolic disorders in the world. It is still a poorly understood human disease that becomes as a major health problem more and more relevant for western society. Obesity is defined as a body weight more than 20% in excess of the ideal body weight, frequently resulting in a significant impairment of health. Obesity may be measured by body mass index, an indicator of adiposity or fatness. Further parameters for defining obesity are waist circumferences, skinfold thickness and bioimpedance. It is associated with an increased risk for cardiovascular disease, hypertension, diabetes mellitus type II, hyperlipidaemia and an increased mortality rate. Obesity is influenced by genetic, metabolic, biochemical, psychological, and behavioral factors and can be caused by different reasons such as non-insulin dependent diabetes, increase in triglycerides, increase in carbohydrate bound energy and low energy expenditure (Kopelman P.G., (2000) Nature 404: 635-643).

The concept of 'metabolic syndrome' (syndrome x, insulin-resistance syndrome, deadly quartet) was first described 1966 by Camus and reintroduced 1988 by Reaven (Camus JP, 1966, Rev Rhum Mal Osteoartic 33: 10-14; Reaven et al. 1988, Diabetes, 37: 1595-1607). Today, metabolic syndrome is commonly defined as clustering of cardiovascular risk factors like hypertension, abdominal obesity, high blood levels of triglycerides and fasting glucose as well as low blood levels of HDL cholesterol. Insulin resistance greatly increases the risk of developing the metabolic syndrome (Reaven, 2002, Circulation 106: 286-288). The metabolic syndrome often precedes the development of type 2 diabetes and cardiovascular disease (Lakka H.M., 2002, JAMA 288: 2709-2716). The control of blood lipid levels and blood glucose levels is essential for the treatment of the metabolic syndrome (see, for example, Santomauro A.T. et al., (1999) Diabetes, 48: 1836-1841).

The molecular factors regulating food intake and body weight balance are incompletely understood. Even if several candidate genes have been described which are supposed to influence the homeostatic system(s) that regulate body mass/weight, like leptin or the peroxisome proliferator-activated receptor-gamma co-activator, the distinct molecular mechanisms and/or molecules influencing obesity or body weight/body mass regulations are not known.

WO 2004/020465 A2 discloses novel uses for energy homeostasis regulating proteins or polynucleotides encoding these in the diagnosis, study, prevention and treatment of metabolic diseases and disorders.

WO 03/072714 A2 relates to the use of proteins comprising at least one follistatin domain to modulate the level or activity of growth and differentiation factor-8 (GDF-8).

WO 03/102163 A2 provides methods for identifying an agent for treating a diabetic or a pre-diabetic individual.

EP 0 869 176 A1 relates to a method for obtaining a novel antigen to which antibodies associated with rheumatoid arthritis (RA) react specifically, and to detecting RA patients by using the antigen, as well as to a composition and a kit for the same.

Schneyer et al. (Molecular and Cellular Endocrinology, vol. 180, pp. 33-38 (2001)) relates to the identification and characterisation of follistatin-related protein (FSRP).

Kawabata et al. (Arthritis and Rheumatism, vol. 50, pp. 660-668, (2004)) describes ameliorative effects of follistatin-related protein/TSC-36/FTSL1 on joint inflammation in a mouse model of arthritis.

There is a need in the prior art for the identification of candidate genes that are specifically expressed in early development in certain pancreatic tissues. These genes and the thereby encoded proteins can provide tools to the diagnosis and treatment of severe pancreatic disorders and related diseases. Therefore, this invention describes secreted proteins that are specifically expressed in pancreatic tissues early in the development. The invention relates to the use of these genes and proteins in the diagnosis, prevention and/or treatment of pancreatic dysfunctions, such as diabetes, and other related diseases such as obesity and/or metabolic syndrome. These proteins and genes are especially useful in regeneration processes, such as regeneration of the pancreas cells.

Herein, we disclose secreted factors referred to as DG153 and DG177 which are involved in pancreas development, regeneration, and in the regulation of energy homeostasis.

The mRNA encoding the low molecular weight DG153 protein was described to be expressed in human liver (see Kawamoto et al., Gene 1996 174: 151-158). The highly conserved arginine-rich protein was shown to be mutated in different cancers, such as renal cell carcinomas, pancreatic, lung, breast, and prostate cancers, and in squamous cell carcinoma of the head and neck (Shridhar et al., Oncogene 12, 1931-1939 (1996), Shridhar et al. Cancer Res. 56, 5576-5578 (1996), Shridhar et al, Oncogene 14, 2213-2216 (1997)). The mutations associated with these cancers appear to be centered in an imperfect trinucleotide repeat that putatively encodes a stretch of 15 to 18 arginines. The association of these mutations with cancer suggests that the putative protein may be a growth factor that plays an important role in the regulation of cell growth and development. No further functional data are available in the scientific prior art for this protein.

However, the DG153 protein and possible further uses in different disease indications are disclosed in several patent applications. For example, DG153 is described as human shear stress response protein useful in the diagnosis, treatment and screening of vascular diseases caused by arteriosclerosis, including heart failure, post-PTCA restenosis and hypertension (see WO 01/25427). Patent application WO 02/74956 describes a 98% identical protein as useful for treating neurodegenerative diseases (such as Parkinson's disease or Alzheimer's diseases). A 97% identical protein is described in WO 02/90541 useful for diagnosing behavioral disorder, or assessing the likelihood of developing behavioral disorder (like Attention Deficit Hyperactivity Disorder or intellectual disorders). The precursor protein (179 amino acids) is described in patent application WO 01/19851 for treating nervous system diseases or disorders (such as Parkinson's disease), and for transplanting cells into nervous system. A 180 amino acid fragment of DG153 is described in WO 01/70174 as being useful for modulating angiogenesis and/or apoptosis for preventing or treating cancer, myocardial infarction and promoting healing, by modulating the activity of vascular endothelial growth factor-modulated gene polypeptide.

DG177 corresponds to human Follistatin-related protein 1 (FLRG), a member of the follistatin family of proteins. Members of this family contain at least one conserved 10-cysteine follistatin domain. In contrast to other members of this family, FLRG (and follistatin) share a common exon/intron domain structure, substantial primary sequence homology, and an ability to irreversibly bind activin. However, the FLRG and follistatin show also structural differences that are responsible for differing functions. Like follistatins, FLRG binds activins, which regulates cell growth and steroidogenesis in the human ovary. Other binding partners of FLRG are bone morphogenetic proteins (BMPs). For example, FLRG proteins downmodulate the effects of activin A and BMP2 on erythroid maturation. In addition, a role of FLRG in the regulation of activin A in brain wound healing was postulated.

The FLRG gene product is thought to be an autoantigen associated with rheumatoid arthritis (WO 97/17441, Tanaka et al., 1998, Int. Immunol. 10, 1305-1314, WO 02/48310A2). FLRG and other genes were found to have polymorphism-associated effects on the suspectibility to low mineral bone density and/or bone damage, and hence osteoporosis (WO 03/054218). The expression of FLRG regulated in specific cancers (see, for example, WO 03/042661, WO 03/027633, WO 02/12440, US2002014298A1, WO 02/059377, WO 02/21996).
D1,D3-D7

To the best of our knowledge, no disclosure has been made in the prior art that describes a function of DG153 or DG177 in pancreatic disorders including diabetes, in obesity or metabolic syndrome. In addition, a role of the secreted factors DG153 or DG177 in the regeneration of tissues such as pancreatic tissues and others is novel and surprising. Thus, since secreted proteins are a major target for drug action and development, it is of high value to identify and characterize novel functions for secreted proteins. The present invention advances the state of the art by providing previously unknown functions for the low molecular weight human secreted protein DG177. This satisfies a need in the art by providing new therapeutic strategies for treating pancreatic diseases (e.g. diabetes), obesity, and/or metabolic syndrome, for example, treating diabetes by regeneration of beta cells. DG177 can be used to promote a regeneration of, for exmple, pancreas cells which then start producing insulin on their own. Therefore, further discovery and development of such novel functions for secreted proteins like DG177 would have a strong beneficial affect on medical services and healthcare.

Herein disclosed are secreted proteins with novel functions in the human metabolism, regeneration and pancreatic developmental processes. Herein disclosed are specific genes and proteins encoded thereby and effectors/modulators thereof involved in the regulation of pancreatic function and metabolism, especially in pancreas diseases such as diabetes mellitus, e.g. insulin dependent diabetes mellitus and/or non-insulin dependent diabetes mellitus, and/or metabolic syndrome, obesity, and/or related disorders such as coronary heart disease, eating disorder, cachexia, hypertension, hypercholesterolemia (dyslipidemia), liver fibrosis, and/or gallstones. Further, herein disclosed are specific genes and proteins encoded thereby and effectors/modulators thereof involved in the modulation, e.g. stimulation, of pancreatic development and/or regeneration of pancreatic cells or tissues, e.g. cells having exocrinous functions such as adnar cells, centroacinar cells and/or ductal cells, and/or cells having endocrinous functions, particularly cells in Langerhans islets such as alpha-, beta-, delta- and/or PP-cells, more particularly beta-cells.

The scope of the invention is defined by the appended claims.

In this invention, we used a screen for secreted factors expressed in developing mammalian (mouse) pancreas, as described in more detail in the Examples section (see Example 1). This screen identified DG153 and DG177 as secreted factors expressed in developing mouse pancreas. The present disclosure describes in particular mammalian DG177 proteins and the polynucleotides encoding these, in particular human as being involved in the conditions and processes mentioned above.

The present disclosure relates to DG177 polynucleotides encoding polypeptides with novel functions in the development and regeneration of pancreatic tissues and thus in mammalian pancreatic diseases (e.g. diabetes), and also in body-weight regulation, energy homeostasis, and obesity, fragments of said polynucleotides, polypeptides encoded by said polynucleotides or fragments thereof. Also disclosed are vectors, host cells, and recombinant methods for producing the polypeptides and polynucleotides of the invention. Also disclosed are effectors/modulators of DG153 or DG177 polynucleotides and/or polypeptides, e.g. antibodies, biologically active nucleic acids, such as antisense molecules, RNAi molecules or ribozymes, aptamers, peptides or low-molecular weight organic compounds recognizing said polynucleotides or polypeptides.

DG177 homologous proteins and nucleic acid molecules coding therefore are obtainable from vertebrate species. Particularly preferred are nucleic adds encoding the human DG177 proteins and variants thereof. The disclosure particularly relates to nucleic add molecules encoding polypeptides contributing to regulating the energy homeostasis and the mammalian metabolism, wherein said nucleic add molecules comprise
(a) the nucleotide sequence of human DG177 (SEQ ID NO: 4) and/or a sequence complementary thereto,
(b) a nucleotide sequence which hybridizes at 50°C in a solution containing 1 x SSC and 0.1 % SDS to a sequence of (a),
(c) a sequence corresponding to the sequences of (a) or (b) within the degeneration of the genetic code,
(d) a sequence which encodes a polypeptide which is at least 85%, preferably at least 90%, more preferably at least 95%, more preferably at least 98% and up to 99,6% identical to an amino acid sequence of the human DG177 protein (SEQ ID NO: 5),

The function of the mammalian DG153 or DG177 in metabolism was validated by analyzing the expression of the transcripts in different tissues and by analyzing the role in adipocyte differentiation.

Expression profiling studies (see Examples for more detail) confirm the particular relevance of DG153 and DG177 as regulators of energy metabolism in mammals.

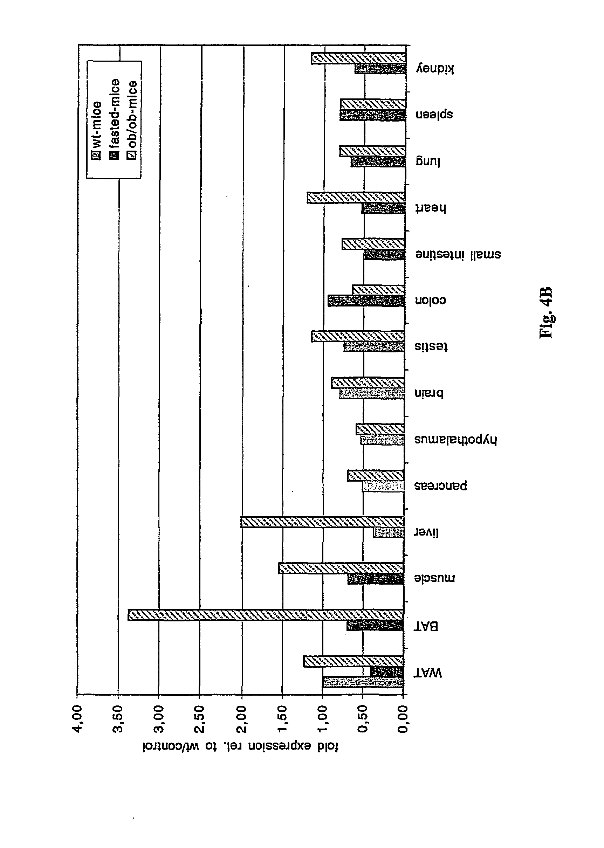

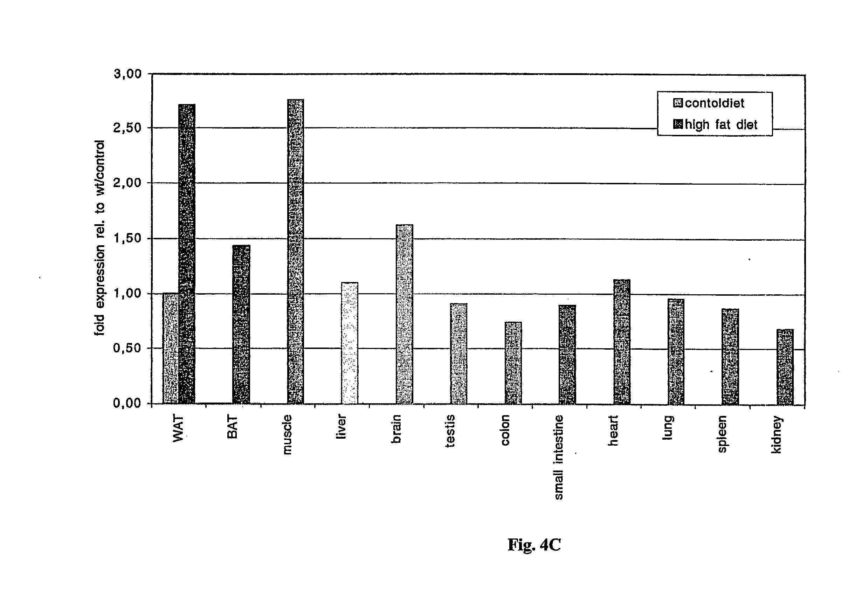

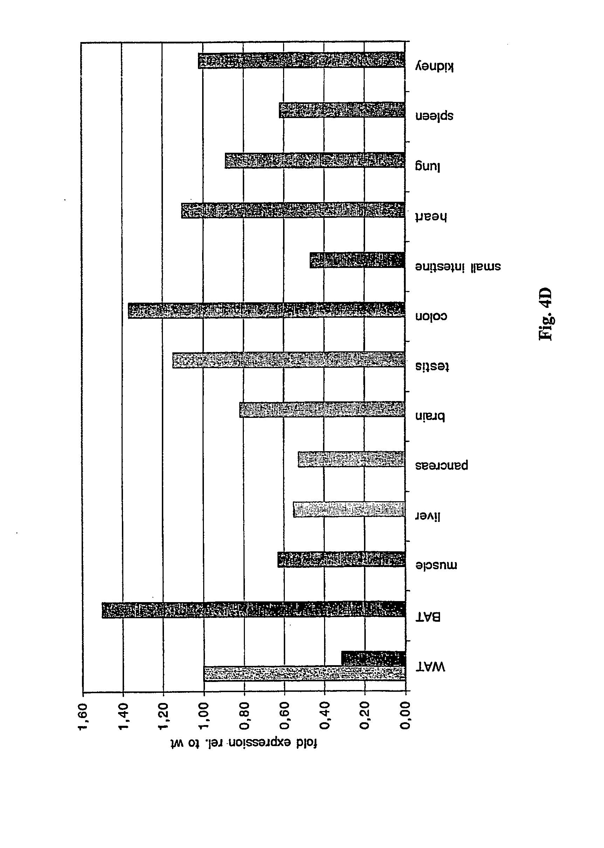

In mouse models of insulin resistance and/or diabetes expression, DG177 is significantly up-regulated in metabolic active tissues (BAT) and muscle which is supporting an essential role of DG177 in the regulation of the mammalian metabolism, particularly in processes related to, obesity, diabetes, or metabolic syndrome (Fig. 4B). We also found, that the expression of DG177 is down-regulated in WAT and liver of fasted wild-type mice, compared to wild type mice fed a standard diet (Fig. 4B). In addition, expression of DG177 mRNA is significantly up-regulated in WAT and muscle in mice showing symptoms of diabetes, lipid accumulation, and high plasma lipid levels, if fed a high fat diet (Fig. 4C). In non-obese diabetic (NOD) mice, expression of DG177 is significantly down-regulated in white adipose tissue compared to wild type mice, as shown in Fig. 4D. Thus, DG177 protein is playing a role in the regulation of metabolism, particularly energy homeostasis and thermogenesis.

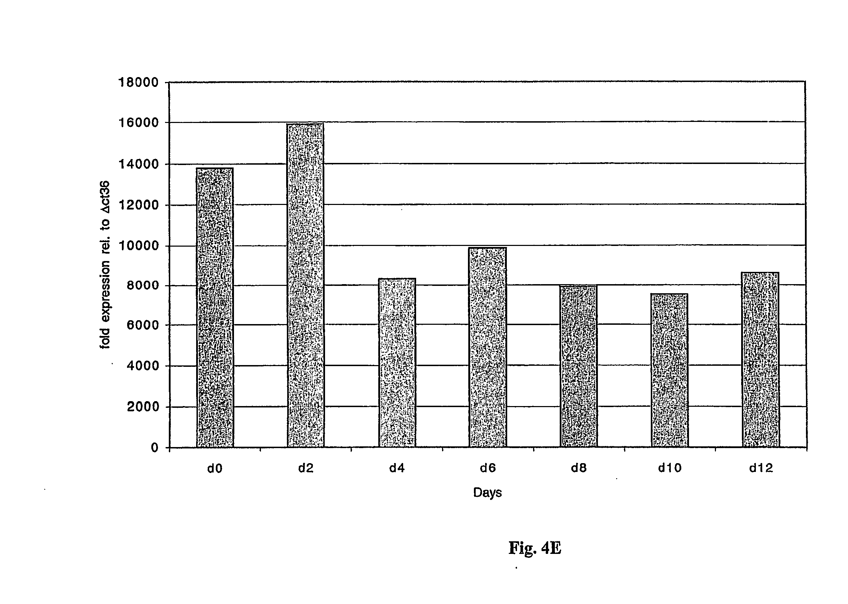

We show (see Examples and Fig. 4E) that the DG177 mRNA is down-regulated during differentiation of preadipocyctes into mature adipocyctes. The results are suggesting a role of DG177 as modulator of adipogenesis.

Microarrays are analytical tools routinely used in bioanalysis. A microarray has molecules distributed over, and stably associated with, the surface of a solid support. The term "microarray" refers to an arrangement of a plurality of polynucleotides, polypeptides, antibodies, or other chemical compounds on a substrate. Microarrays of polypeptides, polynucleotides, and/or antibodies have been developed and find use in a variety of applications, such as monitoring gene expression, drug discovery, gene sequencing, gene mapping, bacterial identification, and combinatorial chemistry. One area in particular in which microarrays find use is in gene expression analysis (see Example 4). Array technology can be used to explore the expression of a single polymorphic gene or the expression profile of a large number of related or unrelated genes. When the expression of a single gene is examined, arrays are employed to detect the expression of a specific gene or its variants. When an expression profile is examined, arrays provide a platform for identifying genes that are tissue specific, are affected by a substance being tested in a toxicology assay, are part of a signaling cascade, carry out housekeeping functions, or are specifically related to a particular genetic predisposition, condition, disease, or disorder.

Microarrays may be prepared, used, and analyzed using methods known in the art (see for example, Brennan, T.M. et al. (1995) U.S. Patent No. 5,474,796; Schena, M. et al. (1996) Proc. Natl. Acad. Sci. 93: 10614-10619; Baldeschweiler et al., PCT application WO 95/251116; Shalon, D. et al., PCT application WO 95/35505; Heller, R.A. et al. (1997) Proc. Natl. Acad. Sci. 94: 2150-2155; Heller, M.J. et al. (1997) U.S. Patent No. 5,605,662). Various types of microarrays are well known and thoroughly described in Schena, M., ed. (1999; DNA Microarrays: A Practical Approach, Oxford University Press, London).

Oligonucleotides or longer fragments derived from any of the polynucleotides described herein may be used as elements on a microarray. The microarray can be used in transcript imaging techniques which monitor the relative expression levels of large numbers of genes simultaneously as described below. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents which are highly effective and display the fewest side effects may be selected for a patient based on his/her pharmacogenomic profile.

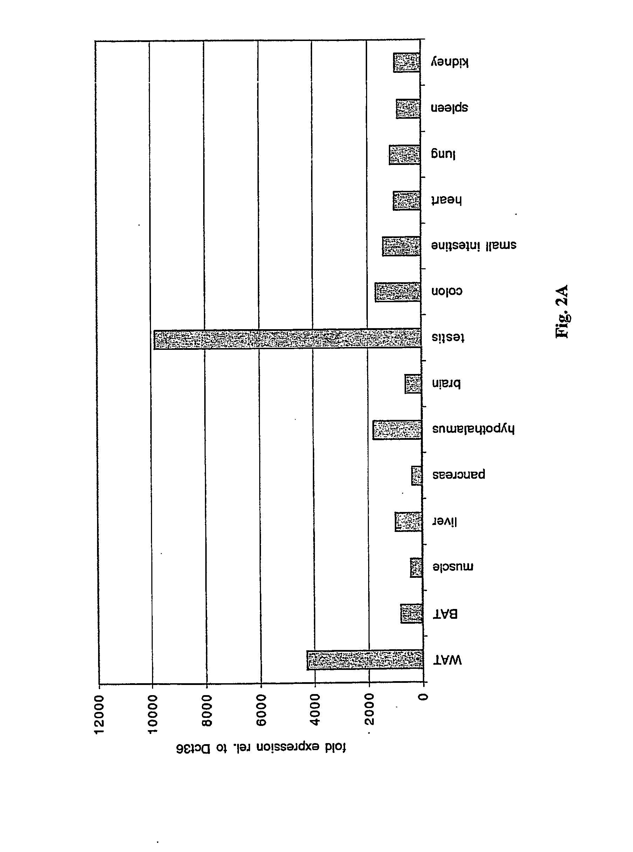

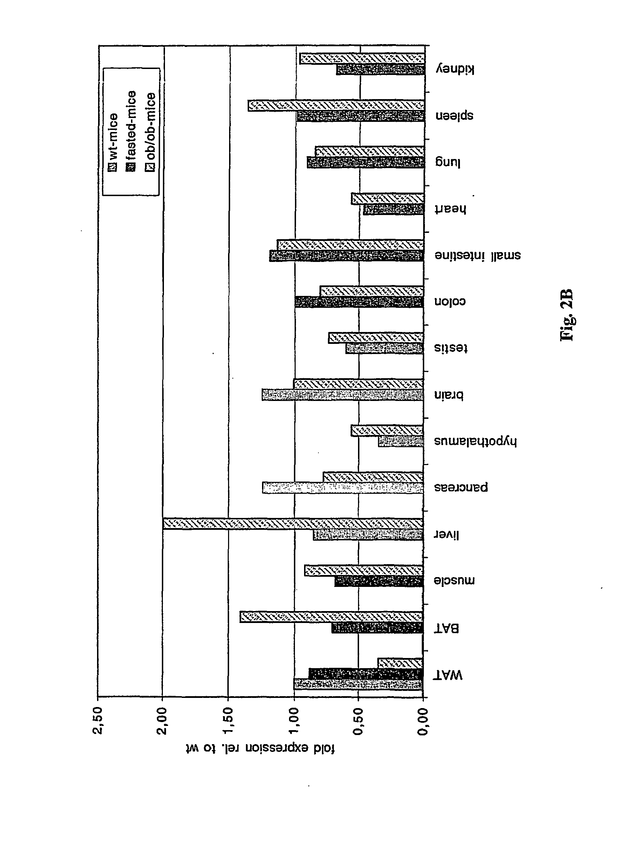

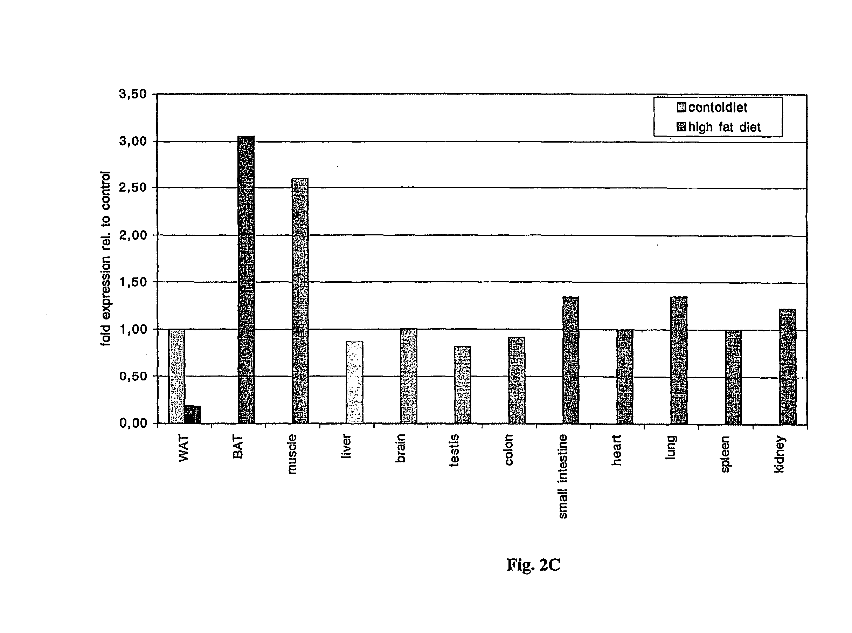

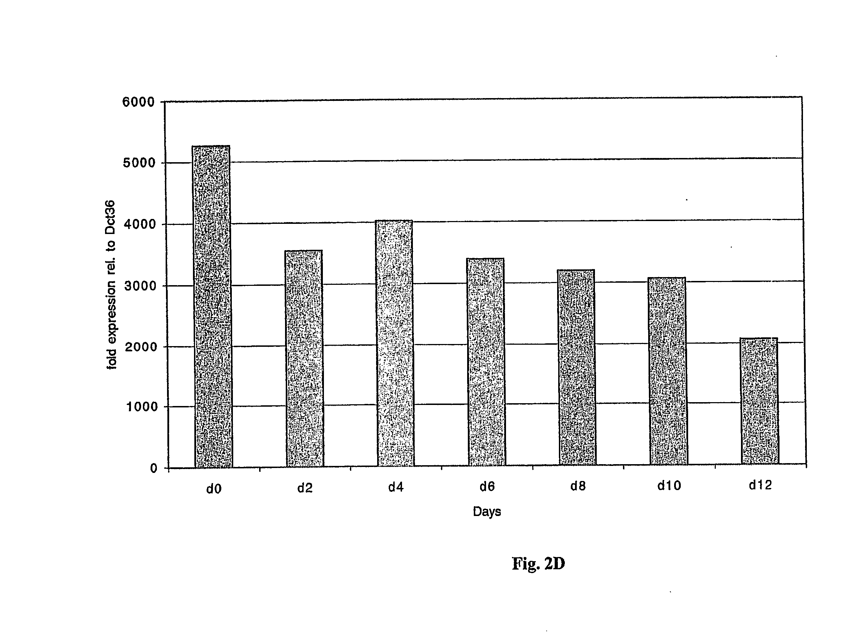

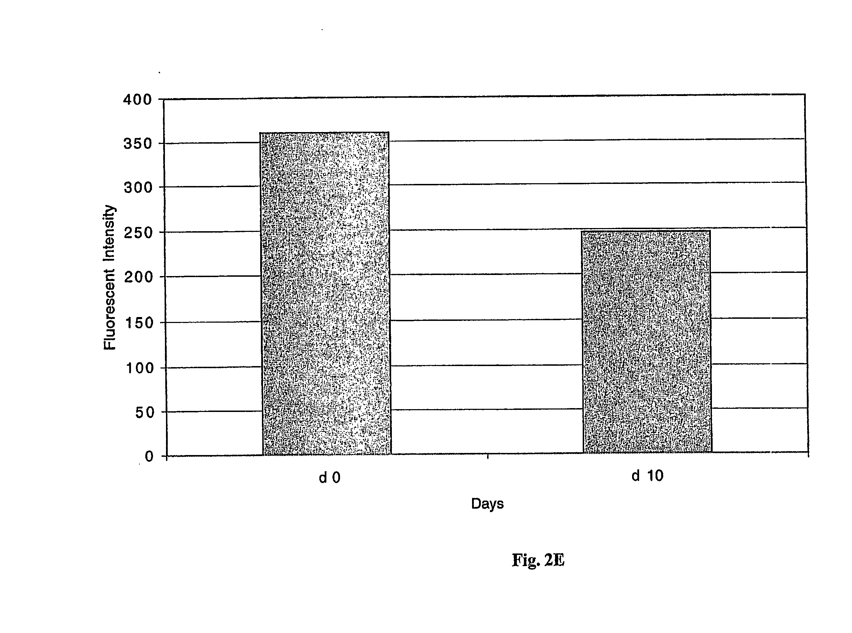

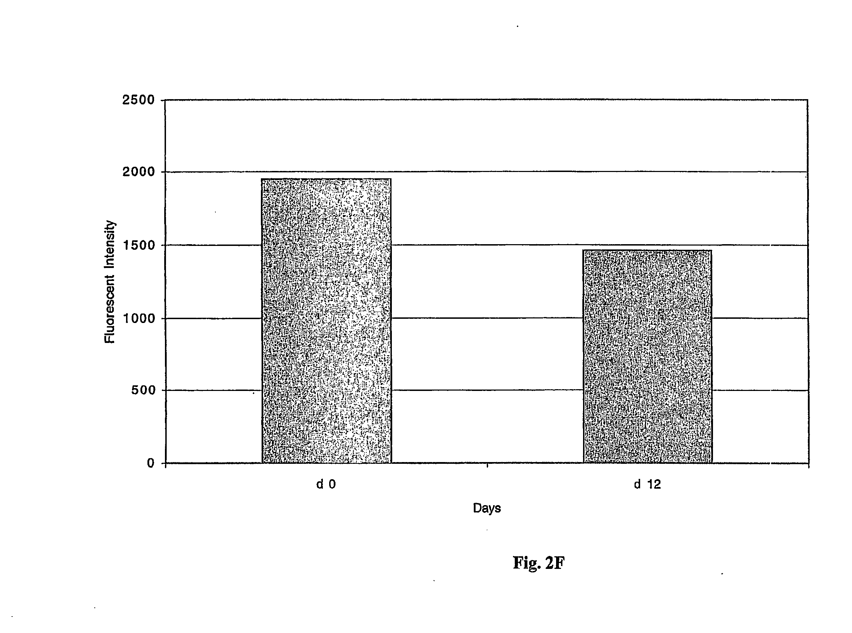

As determined by microarray analysis, DG153 shows differential expression in human primary adipocytes. A downregulation is observed concerning the expression of DG153 is during the murine 3T3-L1 (Fig. 2E) and human adipocyte differentiation (see Fig. 2F). The DG153 protein in preadipocyctes has the potential to inhibit adipose differentiation at a very early stage. Therefore, the DG153 protein might play an essential role in adipogenesis. The results are suggesting a role of DG153 in the regulation in human metabolism, for example, as effector/modulator (for example, inhibitor) of adipogenesis. Thus, DG153 is a strong candidate for the manufacture of a pharmaceutical composition and a medicament for the treatment of conditions related to human metabolism, such as diabetes, obesity, and/or metabolic syndrome.

The disclosure also encompasses polynucleotides that encode the proteins of the invention and homologous proteins. Accordingly, any nucleic acid sequence, which encodes the amino acid sequences of the proteins of the invention and homologous proteins, can be used to generate recombinant molecules that express the proteins of the invention and homologous proteins. In a particular embodiment, the disclosure encompasses nucleic acids encoding DG177. It will be appreciated by those skilled in the art that as a result of the degeneracy of the genetic code, a multitude of nucleotide sequences encoding the proteins, some bearing minimal homology to the nucleotide sequences of any known and naturally occurring gene, may be produced.

Also encompassed by the disclosure are polynucleotide sequences that are capable of hybridizing to the claimed nucleotide sequences, and in particular, those of the polynucleotide encoding the proteins for use according to the invention, under various conditions of stringency. Hybridization conditions are based on the melting temperature (Tm) of the nucleic acid binding complex or probe, as taught in Wahl & Berger (1987: Methods Enzymol. 152: 399-407) and Kimmel (1987; Methods Enzymol. 152: 507-511), and may be used at a defined stringency. Preferably, hybridization under stringent conditions means that after washing for 1 h with 1 x SSC and 0.1 % SDS at 50°C, preferably at 55°C, more preferably at 62°C and most preferably at 65°C, particularly for 1 h in 0.2 x SSC and 0.1% SDS at 50°C, preferably at 55°C, more preferably at 62°C and most preferably at 65°C, a positive hybridization signal is observed. Altered nucleic acid sequences encoding the proteins which are encompassed by the invention include deletions, insertions or substitutions of different nucleotides resulting in a polynucleotide that encodes the same or a functionally equivalent protein.

The encoded proteins may also contain deletions, insertions or substitutions of amino add residues, which produce a silent change and result in functionally equivalent proteins. Deliberate amino acid substitutions may be made on the basis of similarity in polarity, charge, solubility, hydrophobicity, hydrophilicity, and/or the amphipathic nature of the residues as long as the biological activity of the protein is retained. Furthermore, the disclosure relates to peptide fragments of the proteins or derivatives thereof such as cyclic peptides, retro-inverso peptides or peptide mimetics having a length of at least 4, preferably at least 6 and up to 50 amino acids.

Also disclosed are alleles of the genes encoding the proteins of the invention and homologous proteins. As used herein, an 'allele' or 'allelic sequence' is an alternative form of the gene, which may result from at least one mutation in the nucleic acid sequence. Alleles may result in altered mRNAs or polypeptides whose structures or function may or may not be altered. Any given gene may have none, one or many allelic forms.

Common mutational changes, which give rise to alleles, are generally ascribed to natural deletions, additions or substitutions of nucleotides. Each of these types of changes may occur alone or in combination with the others, one or more times in a given sequence.

The nucleic acid sequences encoding DG177 and homologous proteins may be extended utilizing a partial nucleotide sequence and employing various methods known in the art to detect upstream sequences such as promoters and regulatory elements.

In order to express a biologically active protein, the nucleotide sequences encoding the proteins or functional equivalents, may be inserted into appropriate expression vectors, i.e., a vector which contains the necessary elements for the transcription and translation of the inserted coding sequence. Methods, which are well known to those skilled in the art, may be used to construct expression vectors containing sequences encoding the proteins and the appropriate transcriptional and translational control elements. Regulatory elements include for example a promoter, an initiation codon, a stop codon, a mRNA stability regulatory element, and a polyadenylation signal. Expression of a polynucleotide can be assured by (i) constitutive promoters such as the Cytomegalovirus (CMV) promoter/enhancer region, (ii) tissue specific promoters such as the insulin promoter (see, Soria et al., 2000, Diabetes 49: 157), SOX2 gene promotor (see Li et al., (1998) Curr. Biol. 8: 971-974), Msi-1 promotor (see Sakakibara et al., (1997) J. Neuroscience 17: 8300-8312), alpha-cardia myosin heavy chain promotor or human atrial natriuretic factor promotor (Klug et al., (1996) J. Clin. Invest 98: 216-224; Wu et al., (1989) J. Biol. Chem. 264: 6472-6479) or (iii) inducible promoters such as the tetracycline inducible system. Expression vectors can also contain a selection agent or marker gene that confers antibiotic resistance such as the neomycin, hygromycin or puromycin resistance genes. These methods include in vitro recombinant DNA techniques, synthetic techniques, and in vivo genetic recombination. Such techniques are described in Sambrook, J. et al. (1989) Molecular Cloning, A Laboratory Manual, Cold Spring Harbor Press, Plainview, N.Y. and Ausubel, F.M. et al. (1989) Current Protocols in Molecular Biology, John Wiley & Sons, New York, N.Y.

In a further embodiment described herein, natural, modified or recombinant nucleic acid sequences encoding the proteins of the invention and homologous proteins may be ligated to a heterologous sequence to encode a fusion protein.

A variety of expression vector/host systems, as known in the art, may be utilized to contain and express sequences encoding the proteins or fusion proteins. These include, but are not limited to, micro-organisms such as bacteria transformed with recombinant bacteriophage, plasmid or cosmid DNA expression vectors; yeast transformed with yeast expression vectors; insect cell systems infected with virus expression vectors (e.g., baculovirus, adenovirus, adeno-associated virus, lentiverus, retrovirus); plant cell systems transformed with virus expression vectors (e.g., cauliflower mosaic virus, CaMV; tobacco mosaic virus, TMV) or with bacterial expression vectors (e.g., Ti or PBR322 plasmids); or animal cell systems.

The presence of polynucleotide sequences in a sample can be detected by DNA-DNA or DNA-RNA hybridization and/or amplification using probes or portions or fragments of said polynucleotides. Nucleic acid amplification based assays involve the use of oligonucleotides or oligomers based on the sequences specific for the gene to detect transformants containing DNA or RNA encoding the corresponding protein. As used herein 'oligonucleotides' or 'oligomers' refer to a nucleic acid sequence of at least about 10 nucleotides and as many as about 60 nucleotides, preferably about 15 to 30 nucleotides, and more preferably about 20-25 nucleotides, which can be used as a probe or amplimer.

A wide variety of labels and conjugation techniques are known by those skilled in the art and may be used in various nucleic acid and amino add assays. Means for producing labeled hybridization or PCR probes for detecting polynucleotide sequences include oligo-labeling, nick translation, end-labeling of RNA probes, PCR amplification using a labeled nucleotide, or enzymatic synthesis. These procedures may be conducted using a variety of commercially available kits (Pharmacia & Upjohn, (Kalamazoo, Mich.); Promega (Madison Wis.); and U.S. Biochemical Corp., (Cleveland, Ohio).

The presence of DG177 in a sample can be determined by immunological methods or activity measurement. A variety of protocols for detecting and measuring the expression of proteins, using either polyclonal or monoclonal antibodies specific for the protein or reagents for determining protein activity are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on the protein is preferred, but a competitive binding assay may be employed. These and other assays are described, among other places, in Hampton, R. et al. (1990; Serological Methods, a Laboratory Manual, APS Press, St Paul, Minn.) and Maddox, D.E. et al. (1983; J. Exp. Med. 158: 1211-1226).

Suitable reporter molecules or labels, which may be used, include radionuclides, enzymes, fluorescent, chemiluminescent or chromogenic agents as well as substrates, co-factors, inhibitors, magnetic particles, and the like.

Host cells transformed with nucleotide sequences encoding a protein of the invention may be cultured under conditions suitable for the expression and recovery of said protein from cell culture. The protein produced by a recombinant cell may be secreted or contained intracellularly depending on the sequence or/and the vector used. As will be understood by those of skill in the art, expression vectors containing polynucleotides, which encode the protein may be designed to contain signal sequences, which direct secretion of the protein through a prokaryotic or eukaryotic cell membrane. Other recombinant constructions may be used to join sequences encoding the protein to nucleotide sequence encoding a polypeptide domain, which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAG extension/affinity purification system (Immunex Corp., Seattle, Wash.) The inclusion of cleavable linker sequences such as those specific for Factor XA or Enterokinase (Invitrogen, San Diego, Calif.) between the purification domain and the desired protein may be used to facilitate purification.

The data disclosed herein show that the DG177 nucleic acids and proteins are useful in diagnostic and therapeutic applications implicated, pancreatic diseases selected from diabetes (e.g. diabetes mellitus such as insulin dependent diabetes mellitus and/or non insulin dependent diabetes mellitus), obesity, and/or metabolic syndrome. Further, the data show that the DG153 and DG177 nucleic acids and proteins and effector/modulator molecules thereof are useful for the modulation, e.g. stimulation, of pancreatic development and/or for the regeneration of pancreatic cells or tissues, e.g. cells having exocrinous functions such as acinar cells, centroacinar cells and/or ductal cells, and/or cells having endocrinous functions, Particularly cells in Langerhans islets such as alpha-, beta-, delta- and/or PP-cells, more particularly beta-cells. Hence, diagnostic and therapeutic uses for the described nucleic acids and proteins are, for example but not limited to, the following: (i) tissue regeneration in vitro and in vivo (regeneration for all these tissues and cell types composing these tissues and cell types derived from these tissues), (ii) small molecule drug target, (iii) antibody target (therapeutic, diagnostic, drug targeting/cytotoxic antibody), (iv) diagnostic and/or prognostic marker, (v) protein therapy, (vi) gene therapy (gene delivery/gene ablation), and / or (vii) research tools.

For example, but not limited to, cDNAs encoding the disclosed and particularly their human homologues may be useful in stimulating, enhancing or regulating the regeneration of tissues, and the proteins and particularly their human homologues may be useful when administered to a subject in need thereof. The compositions for use according to the present invention will have efficacy for treatment of patients suffering from, pancreatic diseases selected from diabetes obesity, and/or metabolic syndrome as described above.

In one embodiment administration of DG177 nucleic acids and proteins in a pharmaceutical composition to a subject in need thereof, particularly a human patient, leads to an at least partial regeneration of, for example, pancreas cells. The composition will then at least partially restore normal pancreatic function. In one example, these cells are beta cells of the islets which then start producing insulin on their own. After administration of this composition e.g. on a regular basis, the beta cells of a diabetic subject will grow back to approach the normal size and number present in a nondiabetic subject. This effect upon the body reverses the condition of diabetes. As the subject's blood sugar level improves, the dosage administered may be reduced in strength. In at least some cases further administration can be discontinued entirely and the subject continues to produce a normal amount of insulin without further treatment. The subject is thereby not only treated but cured entirely of a diabetic condition. Further, beta cells or precursors thereof may be treated in vitro and implanted or reimplanted into a subject in need thereof. Furthermore, other cells of the pancreas can be regenerated in vivo and/or in vitro to cure a certain condition.

The DG177 nucleic acids and proteins are useful in diagnostic and therapeutic applications implicated in various embodiments as described below. For example, but not limited to, cDNAs encoding the proteins and particularly their human homologues may be useful in gene therapy, and the proteins and particularly their human homologues may be useful when administered to a subject in need thereof. The compositions for use according to the present invention will have efficacy for treatment of patients suffering from, pancreatic diseases selected from diabetes obesity, and/or metabolic syndrome as described above.

The nucleic acids or fragments thereof, may further be useful in diagnostic applications, wherein the presence or amount of the nucleic adds or the proteins are to be assessed.

Herein also disclosed is that antibodies, which are specific for the proteins for use according to the invention and homologous proteins, may be used directly as an effector/modulator, e.g. an antagonist or an agonist, or indirectly as a targeting or delivery mechanism for bringing a pharmaceutical agent to cells or tissue which express the protein. The antibodies may be generated using methods that are well known in the art. Such antibodies may include, but are not limited to, polyclonal, monoclonal, chimeric single chain, Fab fragments, and fragments produced by a Fab expression library. Neutralising antibodies, (i.e., those which inhibit dimer formation) are especially preferred for therapeutic use.

For the production of antibodies, various hosts including goats, rabbits, rats, mice, humans, and others, may be immunized by injection with the protein or any fragment or oligopeptide thereof which has immunogenic properties. Depending on the host species, various adjuvants may be used to increase immunological response. It is preferred that the peptides, fragments or oligopeptides used to induce antibodies to the protein have an amino acid sequence consisting of at least five amino acids, and more preferably at least 10 amino acids.

Monoclonal antibodies to the proteins may be prepared using any technique that provides for the production of antibody molecules by continuous cell lines in culture. These include, but are not limited to, the hybridoma technique, the human B-cell hybridoma technique, and the EBV-hybridoma technique (Köhler, G. and Milstein C., (1975) Nature 256: 495-497; Kozbor, D. et al. (1985) J. Immunol. Methods 81: 31-42; Cote, R.J. et al., (1983) Proc. Natl. Acad. Sci. 80: 2026-2030; Cole, S.P. et al., (1984) Mol. Cell Biochem. 62: 109-120).

In addition, techniques developed for the production of 'chimeric antibodies', the splicing of mouse antibody genes to human antibody genes to obtain a molecule with appropriate antigen specificity and biological activity can be used (Morrison, S.L. et al., (1984) Proc. Natl. Acad. Sci. 81: 6851-6855; Neuberger, M.S. et al (1984) Nature 312: 604-608; Takeda, S. et al. (1985) Nature 314: 452-454). Alternatively, techniques described for the production of single chain antibodies may be adapted, using methods known in the art, to produce single chain antibodies specific for the proteins of the invention and homologous proteins. Antibodies with related specificity, but of distinct idiotypic composition, may be generated by chain shuffling from random combinatorial immunoglobulin libraries (Kang A.S. et al., (1991) Proc. Natl. Acad. Sci. 88: 11120-11123). Antibodies may also be produced by inducing in vivo production in the lymphocyte population or by screening recombinant immunoglobulin libraries or panels of highly specific binding reagents as disclosed in the literature (Orfandi, R., et al. (1989) Proc. Natl. Acad. Sci. 86: 3833-3837; Winter, G. and Milstein C., (1991) Nature 349: 293-299).

Antibody fragments, which contain specific binding sites for the proteins may also be generated. For example, such fragments include, but are not limited to, the F(ab')₂ fragments which can be produced by Pepsin digestion of the antibody molecule and the Fab fragments which can be generated by reducing the disulfide bridges of F(ab')₂ fragments. Alternatively, Fab expression libraries may be constructed to allow rapid and easy identification of monoclonal Fab fragments with the desired specificity (Huse, W.D. et al. (1989) Science 246: 1275-1281).

Various immunoassays may be used for screening to identify antibodies having the desired specificity. Numerous protocols for competitive binding and immunoradiometric assays using either polyclonal or monoclonal antibodies with established specificities are well known in the art. Such immunoassays typically involve the measurement of complex formation between the protein and its specific antibody. A two-site, monoclonal-based immunoassay utilizing monoclonal antibodies reactive to two non-interfering protein epitopes are preferred, but a competitive binding assay may also be employed (Maddox, supra).

Also disclosed is that polynucleotides or fragments thereof or nucleic acid effector/modulator molecules such as antisense molecules, aptamers, RNAi molecules or ribozymes may be used for therapeutic purposes. Aptamers, i.e. nucleic acid molecules, which are capable of binding to a protein described herein and modulating its activity, may be generated by a screening and selection procedure involving the use of combinatorial nucleic acid libraries.

Also disclosed is that antisense molecules may be used in situations in which it would be desirable to block the transcription of the mRNA. Cells may be transformed with sequences complementary to polynucleotides encoding DG153 or DG177 and/or homologous proteins. Thus, antisense molecules may be used to modulate/effect protein activity or to achieve regulation of gene function. Such technology is now well known in the art, and sense or antisense oligomers or larger fragments, can be designed from various locations along the coding or control regions of sequences encoding the proteins. Expression vectors derived from retroviruses, adenovirus, herpes or vaccinia viruses or from various bacterial plasmids may be used for delivery of nucleotide sequences to the targeted organ, tissue or cell population. Methods, which are well known to those skilled in the art, can be used to construct recombinant vectors, which will express antisense molecules complementary to the polynucleotides of the genes encoding the proteins of the invention and homologous proteins. These techniques are described both in Sambrook et al. (supra) and in Ausubel et al. (supra). Genes encoding the proteins disclosed herein can be turned off by transforming a cell or tissue with expression vectors, which express high levels of polynucleotides that encode the proteins disclosed herein or fragments thereof. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

As mentioned above, modifications of gene expression can be obtained by designing antisense molecules, e.g. DNA, RNA or nucleic acid analogues such as PNA, to the control regions of the genes encoding DG153 or DG177 and/or homologous proteins, i.e., the promoters, enhancers, and introns. Oligonucleotides derived from the transcription initiation site, e.g., between positions -10 and +10 from the start site, are preferred. Similarly, inhibition can be achieved using "triple helix" base-pairing methodology. Triple helix pairing is useful because it cause inhibition of the ability of the double helix to open sufficiently for the binding of polymerases, transcription factors or regulatory molecules. Recent therapeutic advances using triplex DNA have been described in the literature (Gee, J.E. et al. (1994) Gene 149: 109-114; Huber, B.E. and Carr B.I., Molecular and Immunologic Approaches, Futura Publishing Co., Mt. Kisco, N.Y.). The antisense molecules may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. Examples, which may be used, include engineered hammerhead motif ribozyme molecules that can be specifically and efficiently catalyze endonucleolytic cleavage of sequences encoding the proteins of the invention and homologous proteins. Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridization with complementary oligonucleotides using ribonuclease protection assays.

Nucleic acid effector/modulator molecules, e.g. antisense molecules and ribozymes may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences. Such DNA sequences may be incorporated into a variety of vectors with suitable RNA polymerase promoters such as T7 or SP6. Alternatively, these cDNA constructs that synthesize antisense RNA constitutively or inducibly can be introduced into cell lines, cells or tissues. RNA molecules may be modified to increase intracellular stability and half-life. Possible modifications include, but are not limited to, the addition of flanking sequences at the 5' and/or 3' ends of the molecule or modifications in the nucleobase, sugar and/or phosphate moieties, e.g. the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection and by liposome injections may be achieved using methods, which are well known in the art. Any of the therapeutic methods described above may be applied to any suitable subject including, for example, mammals such as dogs, cats, cows, horses, rabbits, monkeys, and most preferably, humans.

An additional embodiment described herein relates to the administration of a pharmaceutical composition, in conjunction with a pharmaceutically acceptable carrier, for any of the therapeutic effects discussed above. Such pharmaceutical compositions may consist of DG177 nucleic acids and the proteins and homologous nucleic acids or proteins. The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone or in combination with other agents, drugs or hormones. The pharmaceutical compositions utilized in this invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual or rectal means.

In addition to the active ingredients, these pharmaceutical compositions may contain suitable pharmaceutically-acceptable carriers comprising excipients and auxiliaries, which facilitate processing of the active compounds into preparations, which can be used pharmaceutically. Further details on techniques for formulation and administration may be found in the latest edition of Remington's Pharmaceutical Sciences (Maack Publishing Co., Easton, Pa.).

Pharmaceutical compositions suitable for use in the invention include compositions wherein the active ingredients are contained in an effective amount to achieve the intended purpose. The determination of an effective dose is well within the capability of those skilled in the art. For any compounds, the therapeutically effective dose can be estimated initially either in cell culture assays, e.g., of preadipocyte cell lines or in animal models, usually mice, rabbits, dogs or pigs. The animal model may also be used to determine the appropriate concentration range and route of administration. Such information can then be used to determine useful doses and routes for administration in humans. A therapeutically effective dose refers to that amount of active ingredient, for example the DG177 nucleic acids or proteins or fragments thereof, which is sufficient for treating a specific condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutical compositions, which exhibit large therapeutic indices, are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage from employed, sensitivity of the patient, and the route of administration. The exact dosage will be determined by the practitioner, in light of factors related to the subject that requires treatment. Dosage and administration are adjusted to provide sufficient levels of the active moiety or to maintain the desired effect. Factors, which may be taken into account, include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week or once every two weeks depending on half-life and clearance rate of the particular formulation. Normal dosage amounts may vary from 0.1 to 100,000 microg, up to a total dose of about 1 g, depending upon the route of administration. Guidance as to particular dosages and methods of delivery is provided in the literature and generally available to practitioners in the art. Those skilled in the art employ different formulations for nucleotides than for proteins or their inhibitors. Similarly, delivery of polynucleotides or polypeptides will be specific to particular cells, conditions, locations, etc.

Also disclosed is that antibodies which specifically bind to proteins may be used for the diagnosis of conditions or diseases characterized by or associated with over- or under-expression of the proteins disclosed herein or in assays to monitor patients being treated with the proteins disclosed herein, or effectors / modulators thereof, e.g. agonists, antagonists, or inhibitors. Diagnostic assays include methods which utilize the antibody and a label to detect the protein in human body fluids or extracts of cells or tissues. The antibodies may be used with or without modification, and may be labeled by joining them, either covalently or non-covalently, with a reporter molecule. A wide variety of reporter molecules, which are known in the art may be used several of which are described above.

A variety of protocols including ELISA, RIA, and FACS for measuring proteins are known in the art and provide a basis for diagnosing altered or abnormal levels of gene expression. Normal or standard values for gene expression are established by combining body fluids or cell extracts taken from normal mammalian subjects, preferably human, with antibodies to the protein under conditions suitable for complex formation. The amount of standard complex formation may be quantified by various methods, but preferably by photometric means. Quantities of protein expressed in control and disease, samples e.g. from biopsied tissues are compared with the standard values. Deviation between standard and subject values establishes the parameters for diagnosing disease.

The polynucleotides specific for the DG177 proteins and homologous proteins may be used for diagnostic purposes. The polynucleotides, which may be used, include oligonucleotide sequences, antisense RNA and DNA molecules, and PNAs. The polynucleotides may be used to detect and quantitate gene expression in biopsied tissues in which gene expression may be correlated with disease. The diagnostic assay may be used to distinguish between absence, presence, and excess gene expression, and to monitor regulation of protein levels during therapeutic intervention.

Hybridization with probes which are capable of detecting polynucleotide sequences, including genomic sequences, encoding the proteins disclosed herein or closely related molecules, may be used to identify nucleic acid sequences which encode the respective protein. The hybridization probes of the subject invention may be DNA or RNA and are preferably derived from the nucleotide sequence of the polynucleotide encoding the proteins disclosed herein or from a genomic sequence including promoter, enhancer elements, and introns of the naturally occurring gene. Hybridization probes may be labeled by a variety of reporter groups, for example, radionuclides such as ³²P or ³⁵S or enzymatic labels, such as alkaline phosphatase coupled to the probe via avidin/biotin coupling systems, and the like.

Polynucleotide sequences specific for DG177 proteins and homologous nucleic acids may be used for the diagnosis of conditions or diseases, which are associated with the expression of the proteins. Examples of such diseases include the pancreatic diseases (e.g. diabetes), obesity, metabolic syndrome, and/or others. Polynucleotide sequences specific for the DG177 proteins and homologous proteins may also be used to monitor the progress of patients receiving treatment for pancreatic diseases (e.g. diabetes), obesity, and/or metabolic syndrome. The polynucleotide sequences may be used qualitative or quantitative assays, e.g. in Southern or Northern analysis, dot blot or other membrane-based technologies; in PCR technologies; or in dip stick, pin, ELISA or chip assays utilizing fluids or tissues from patient biopsies to detect altered gene expression.

DG177 nucleotide sequences may be useful in assays that detect activation or induction of various metabolic diseases or dysfunctions. The nucleotide sequences may be labeled by standard methods, and added to a fluid or tissue sample from a patient under conditions suitable for the formation of hybridization complexes. After a suitable incubation period, the sample is washed and the signal is quantitated and compared with a standard value. The presence of altered levels of nucleotide sequences encoding the proteins disclosed herein in the sample indicates the presence of the associated disease. Such assays may also be used to evaluate the efficacy of a particular therapeutic treatment regimen in animal studies, in clinical trials or in monitoring the treatment of an individual patient.

In order to provide a basis for the diagnosis of a disease associated with expression of the DG177 proteins and homologous proteins, a normal or standard profile for expression is established. This may be accomplished by combining body fluids or cell extracts taken from normal subjects, either animal or human, with a sequence or a fragment thereof, which is specific for the nucleic acids encoding the proteins of the invention and homologous nucleic acids, under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for disease. Deviation between standard and subject values is used to establish the presence of disease. Once disease is established and a treatment protocol is initiated, hybridization assays may be repeated on a regular basis to evaluate whether the level of expression in the patient begins to approximate that, which is observed in the normal patient. The results obtained from successive assays may be used to show the efficacy of treatment over a period ranging from several days to months.

With respect to pancreatic diseases (e.g. diabetes), obesity, and/or metabolic syndrome, the presence of an unusual amount of transcript in biopsied tissue from an individual may indicate a predisposion for the development of the disease or may provide a means for detecting the disease prior to the appearance of actual clinical symptoms. A more definitive diagnosis of this type may allow health professionals to employ preventative measures or aggressive treatment earlier thereby preventing the development or further progression of the metabolic diseases and disorders.

Additional diagnostic uses for oligonucleotides designed from the sequences encoding the proteins of the invention and homologous proteins may involve the use of PCR. Such oligomers may be chemically synthesized, generated enzymatically or produced from a recombinant source. Oligomers will preferably consist of two nucleotide sequences, one with sense orientation (5prime.fwdarw.3prime) and another with antisense (3prime.rarw.5prime), employed under optimized conditions for identification of a specific gene or condition. The same two oligomers, nested sets of oligomers or even a degenerate pool of oligomers may be employed under less stringent conditions for detection and/or quantification of closely related DNA or RNA sequences.

The nucleic acid sequences may also be used to generate hybridization probes, which are useful for mapping the naturally occurring genomic sequence. The sequences may be mapped to a particular chromosome or to a specific region of the chromosome using well-known techniques. Such techniques include FISH, FACS or artificial chromosome constructions, such as yeast artificial chromosomes, bacterial artificial chromosomes, bacterial P1 constructions or single chromosome cDNA libraries as reviewed in Price, C. M. (1993) Blood Rev. 7:127-134, and Trask, B. J. (1991) Trends Genet. 7:149-154. FISH (as described in Verma et al. (1988) Human Chromosomes: A Manual of Basic Techniques, Pergamon Press, New York, N.Y.). The results may be correlated with other physical chromosome mapping techniques and genetic map data. Examples of genetic map data can be found in the 1994 Genome Issue of Science (265: 1981f). Correlation between the location of the gene encoding the proteins disclosed herein on a physical chromosomal map and a specific disease or predisposition to a specific disease, may help to delimit the region of DNA associated with that genetic disease.

The nucleotide sequences disclosed herein may be used to detect differences in gene sequences between normal, carrier or affected individuals. An analysis of polymorphisms, e.g. single nucleotide polymorphisms may be carried out. Further, in situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11 q22-23 (Gatti, R. A. et al. (1988) Nature 336: 577-580), any sequences mapping to that area may represent associated or regulatory genes for further investigation. The nucleotide sequences disclosed herein may also be used to detect differences in the chromosomal location due to translocation, inversion, etc. among normal, carrier or affected individuals.

In another embodiment, the proteins for use according to the invention, their catalytic or immunogenic fragments or oligopeptides thereof, an in vitro model, a genetically altered cell or animal, can be used for screening libraries of compounds in any of a variety of drug screening techniques. One can identify effectors, e.g. receptors, enzymes, proteins, ligands, or substrates that bind to, modulate or mimic the action of one or more of the DG177 proteins of the invention. The protein or fragment thereof employed in such screening may be free in solution, affixed to a solid support, borne on a cell surface, or located intracellulary. The formation of binding complexes, between the DG177 proteins of the invention and the agent tested, may be measured. Agents could also, either directly or indirectly, influence the activity of the proteins of the invention.

In addition activity of the proteins disclosed herein against their physiological substrate(s) or derivatives thereof could be measured in cell-based or cell-free assays. Agents may also interfere with posttranslational medications of the protein, such as phosphorylation and dephosphorylation, farnesylation, palmitoylation, acetylation, alkylation, ubiquitination, proteolytic processing, subcellular localization and degradation. Moreover, agents could influence the dimerization or oligomerization of the proteins or, in a heterologous manner, of the proteins with other proteins, for example, but not exclusively, docking proteins, enzymes, receptors, or translation factors. Agents could also act on the physical interaction of the proteins of this invention with other proteins, which are required for protein function, for example, but not exclusively, their downstream signaling.

Methods for determining protein-protein interaction are well known in the art. For example binding of a fluorescently labeled peptide derived from the interacting protein to the DG177 protein or vice versa, could be detected by a change in polarisation. In case that both binding partners, which can be either the full length proteins as well as one binding partner as the full length protein and the other just represented as a peptide are fluorescently labeled, binding could be detected by fluorescence energy transfer (FRET) from one fluorophore to the other. In addition, a variety of commercially available assay principles suitable for detection of protein-protein Interaction are well known In the art, for example but not exclusively AlphaScreen (PerkinElmer) or Scintillation Proximity Assays (SPA) by Amersham. Alternatively, the interaction of the DG153 or DG177 proteins with cellular proteins could be the basis for a cell-based screening assay, in which both proteins are fluorescently labeled and interaction of both proteins is detected by analysing cotranslocation of both proteins with a cellular imaging reader, as has been developed for example, but not exclusively, by Cellomics or EvotecOAI. In all cases the two or more binding partners can be different proteins with one being the protein disclosed herein or in case of dimerization and/or oligomerization the protein itself.

Of particular interest are screening assays for agents that have a low toxicity for mammalian cells. The term "agent" as used herein describes any molecule, e.g. protein or pharmaceutical, with the capability of altering or mimicking the physiological function of one or more of the proteins disclosed herein Candidate agents encompass numerous chemical classes, though typically they are organic molecules, preferably small organic compounds having a molecular weight of more than 50 and less than about 2,500 Daftons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Candidate agents are also found among biomolecules including peptides, saccharides, fatty adds, steroids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Where the screening assay is a binding assay, one or more of the molecules may be joined to a label, where the label can directly or indirectly provide a detectable signal.

Another technique for drug screening, which may be used, provides for high throughput screening of compounds having suitable binding affinity to the protein of interest as described in published PCT application WO 84/03564. In this method, as applied to the proteins disclosed herein large numbers of different small test compounds, e.g. aptamers, peptides, low-molecular weight compounds etc., are provided or synthesized on a solid substrate, such as plastic pins or some other surface. The test compounds are reacted with the proteins or fragments thereof, and washed. Bound proteins are then detected by methods well known in the art. Purified proteins can also be coated directly onto plates for use in the aforementioned drug screening techniques. Alternatively, non-neutralizing antibodies can be used to capture the peptide and immobilize it on a solid support. In another embodiment, one may use competitive drug screening assays in which neutralizing antibodies capable of binding the protein specifically compete with a test compound for binding the protein. In this manner, the antibodies can be used to detect the presence of any peptide, which shares one or more antigenic determinants with the protein.

Compounds that bind DG177 proteins, e.g. antibodies, are useful for the identification or enrichment of cells, which are positive for the expression of the proteins disclosed herein from complex cell mixtures. Such cell populations are useful in transplantation, for experimental evaluation, and as source of lineage and cell specific products, including mRNA species useful in identifying genes specifically expressed in these cells, and as target for the identification of factors of molecules that can affect them. Cells expressing the protein or which have been treated with the protein are useful in transplantation to provide a recipient with pancreatic islet cells, including insulin producing beta cells; for drug screening; experimental models of islet differentiation and interaction with other cell types; in vitro screening assays to define growth and differentiation factors, and to additionally characterize genes involved in islet development and regulation; and the like. The native cells may be used for these purposes, or they may be genetically modified to provide altered capabilities. Cells from a regenerating pancreas, from embryonic foregut, stomach and duodenum, or other sources of pancreatic progenitor cells may be used as a starting population. The progenitor cells may be obtained from any mammalian species, e.g. equine, bovine, porcine, canine, feline, rodent, e.g. mice, rats, hamster, primate, etc. particularly human.

In a high-throughput screening method, the cells are transfected with a DNA construct, e.g. a viral or non-viral vector containing a reporter gene, e.g. the lacZ gene or the GFP gene, under regulatory control of a promoter of a gene involved in for example beta-cell differentiation, e.g. a promoter of a gene stimulation beta-cell differentiation, preferably a Pax4 promoter. The transfected cells are divided into aliquots and each aliquot is contacted with a test substance, e.g., candidate 1, candidate 2 and candidate 3. The activity of the reporter gene corresponds to the capability of the test compound to induce beta-cell differentiation.

Also disclosed is a method which may be combined with the high-through put screening as described above, in which a medium throughput validation is carried out. Therein, the test compound is added to stem cells being cultivated and the insulin production is determined. Following an initial high throughput assay, such as the cell based assay outlined above where for example a Pax4 promoter is used as marker for beta-cell regeneration, the activity of candidate molecules to induce beta-cell differentiation is tested in a validation assay comprising adding said compounds to the culture media of the embryoid bodies. Differentiation into insulin-producing cells is then evaluated, e.g. by comparison to wild type and/or Pax4 expressing ES cells to assess the effectiveness of a compound.

The nucleic acids encoding the DG177 proteins can be used to generate transgenic cell lines and animals. These transgenic non-human animals are useful in the study of the function and regulation of the proteins of the invention in vivo. Transgenic animals, particularly mammalian transgenic animals, can serve as a model system for the investigation of many developmental and cellular processes common to humans. A variety of non-human models of metabolic disorders can be used to test modulators of the protein disclosed herein Misexpression (for example, over-expression or lack of expression) of the protein particular feeding conditions, and/or administration of biologically active compounds can create models of metabolic disorders.

Such assays may use mouse models of insulin resistance and/or diabetes, such as mice carrying gene knockouts in the leptin pathway (for example, ob (leptin) or db (leptin receptor) mice), as described above. In addition to testing the expression of the proteins in such mouse strains (see Examples), these mice could be used to test whether administration of a candidate modulator alters for example lipid accumulation in the liver, in plasma, or adipose tissues using standard assays well known in the art, such as FPLC, colorimetric assays, blood glucose level tests, insulin tolerance tests and others.

Transgenic animals may be made through homologous recombination in non-human embryonic stem cells, where the normal locus of the gene encoding the protein is altered. Alternatively, a nucleic acid construct encoding a protein disclosed herein is injected into oocytes and is randomly integrated into the genome. Vectors for stable integration include plasmids, retroviruses and other animal viruses, yeast artificial chromosomes (YACs), and the like. The modified cells or animals are useful in the study of the function and regulation of the protein of the invention. For example, a series of small deletions and/or substitutions may be made in the gene that encodes the protein to determine the role of particular domains of the protein, functions in pancreatic differentiation, etc.

Furthermore, variants of the described genes like specific constructs of interest include anti-sense molecules, which will block the expression of the protein of the invention, or expression of dominant negative mutations. A detectable marker, such as lac Z or luciferase may be introduced into the locus of the genes of the invention, where up-regulation of expression of the genes of the invention will result in an easily detected change in phenotype.

One may also provide for expression of the genes or variants thereof in cells or tissues where it is not normally expressed or at abnormal times of development In addition, by providing expression of the protein in cells in which they are not normally produced, one can induce changes in cell behavior.

DNA constructs for homologous recombination will comprise at least portions of the genes of the invention with the desired genetic modification, and will include regions of homology to the target locus. DNA constructs for random integration do not need to contain regions of homology to mediate recombination. Conveniently, markers for positive and negative selection are included. DNA constructs for random integration will consist of the nucleic adds encoding the proteins of the invention, a regulatory element (promoter), an intron and a poly-adenylation signal. Methods for generating cells having targeted gene modfications through homologous recombination are known in the art. For non-human embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF).

When non-human ES or embryonic cells or somatic pluripotent stem cells have been transfected, they may be used to produce transgenic animals. After transfection, the cells are plated onto a feeder layer in an appropriate medium. Cells containing the construct may be selected by employing a selective medium. After sufficient time for colonies to grow, they are picked and analyzed for the occurrence of homologous recombination or integration of the construct. Those colonies that are positive may then be used for embryo transfection and morula aggregation. Briefly, morulae are obtained from 4 to 6 weeks old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or in vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the proteins disclosed herein in vivo.

Also disclosed is a kit comprising at least one of
(a) a nucleic add molecule coding for a protein disclosed herein or a functional fragment thereof;
(b) a protein disclosed herein or a fragment or an isoform thereof;
(c) a vector comprising the nucleic acid of (a);
(d) a host cell comprising the nucleic acid of (a) or the vector of (c);
(e) a polypeptide encoded by the nucleic acid of (a);
(f) a fusion polypeptide encoded by the nucleic acid of (a);
(g) an antibody, an aptamer or another effector/modulator against the nucleic acid of (a) or the polypeptide of (b), (e) or (f) and
(h) an anti-sense oligonucleotide of the nucleic add of (a).

The kit may be used for diagnostic or therapeutic purposes or for screening applications as described above. The kit may further contain user instructions.

The Figures show:
Fig. 1 shows human DG153 nucleic acid and proteins.
   Fig. 1A shows the nucleic add sequence of human DG153 protein (SEQ ID NO: 1; GenBank Accession Number M83751).
   Fig. 1B shows the amino acid sequence (one-letter code) of human DG153 protein, longer variant (SEQ ID NO: 2; GenBank Accession Number AAB08753).
   Fig. 1C shows the amino acid sequence (one-letter code) of human DG153 protein, shorter variant (SEQ ID NO: 3; Swiss-Prot Accession Number P55145).
Fig. 2 shows the analysis of DG153 protein expression in mammalian tissues and cells. The relative RNA-expression is shown on the Y-axis, in Fig. 2A to 2C, the tissues tested are given on the X-axis. WAT refers to white adipose tissue, BAT refers to brown adipose tissue. In Fig. 2D to 2F, the X-axis represents the time axis. 'd0' refers to day 0 (start of the experiment), "d10" refers to day 10 of adipocyte differentiation, "d12" refers to day 12 of adipocyte differentiation).
   Fig. 2A shows the quantitative (real-time PCR) analysis of DG153 expression in mouse wild-type tissues.
   Fig. 2B shows the quantitative (real-time PCR) analysis of DG153 expression in genetically obese mice (ob/ob-mice) and fasted mice (fasted-mice) compared to wild-type mice (wt-mice).
   Fig. 2C shows the quantitative (real-time PCR) analysis of DG153 expression in mice fed with a high fat diet compared to mice fed with a control diet.
   Fig. 2D shows the quantitative (real-time PCR) analysis of DG153 expression in mouse fibroblast (3T3-L1) cells during the differentiation from preadipocytes to mature adipocytes.
   Fig. 2E shows the microarray analysis of DG153 expression in mouse fibroblast (3T3-L1) cells during the differentiation from preadipocytes to mature adipocytes.
   Fig. 2F shows the microarray analysis of DG153 expression in human abdominal adipocyte cells, during the differentiation from preadipocytes to mature adipocytes.
Fig. 3 shows human DG177 nucleic acid and protein.
   Fig. 3A shows the nucleic acid sequence of human DG177 protein (SEQ ID NO: 4; GenBank Accession Number NM_007085).
   Fig. 3B shows the amino acid sequence (one-letter code) of human DG177 protein (SEQ ID NO: 5; GenBank Accession Number NP_009016).
Fig. 4 shows the quantitative (real-time PCR) analysis of DG177 protein expression in mammalian tissues and cells. The relative RNA-expression is shown on the Y-axis, in Fig. 4A to 4D the tissues tested are given on the X-axis. WAT refers to white adipose tissue, BAT refers to brown adipose tissue. In Fig. 4E, the X-axis represents the time axis. 'd0' refers to day 0 (start of the experiment), 'd2' - 'd10' refers to day 2 - day 10 of adipocyte differentiation).
   Fig. 4A shows the analysis of DG177 expression in wild-type mouse tissues. Fig. 4B shows the analysis of DG177 expression in genetically obese mice (ob/ob-mice) and fasted mice (fasted-mice) compared to wild-type mice (wt-mice).
   Fig. 4C shows the analysis of DG177 expression in mice fed with a high fat diet compared to mice fed with a control diet.
   Fig. 4D shows the analysis of DG177 expression in mouse tissues from non-obese-diabetic (NOD) mice compared to wild-type mice.
   Fig. 4E shows the analysis of DG177 expression in mammalian fibroblast (3T3-L1) cells during the differentiation from preadipocytes to mature adipocytes.

The examples illustrate the invention:

### Example 1: Identification of secreted factors expressed in pancreas

A screen for secreted factors expressed in developing mouse pancreas was carried out according to methods known by those skilled in the art (see, for example Pera E.M. and De Robertis E.M., (2000) Mech Dev 96: 183-195) with several modifications.

### Expression cDNA library:

During organogenesis, the pancreatic bud is surrounded and influenced by the associated mesenchyme. (see for example, Madsen O.D. et al., (1996) Eur. J. Biochem. 242: 435-445 and Slack, J.M., (1995) Development 121: 1569-1580). Recently, it was suggested, that white adipocytes origin directly from mesenchymal cells (Atanossova P.K., (2003) Folia Med. 45: 41-45). During embryogenesis, the innervation and vascularization of the pancreas can be observed. Therefore, the tissue used in the screen might have contained besides pancreatic cells some adipocyte precursors, blood vessels, as well as neuronal cells.

A mouse embryonic stage 9.5-15 pancreatic bud library was prepared in pCMVSPORT-6 vector using SUPERSCRIPT Plasmid System from Invitrogen according to the manufacturer's instructions. The non-amplified library was electroporated into MaxEff DH10B cells (Invitrogen).

### Secretion cloning

Bacterial clones were picked with sterile toothpicks from agar plates and cultured in 96-deep-well microtiter plates in LB-ampicillin (see Sambrook et al., supra). Aliquots of 8 cultures were pooled, and plasmid DNA was isolated using the BioRobot_9600 apparatus according to the manufacturer's instructions (Qiagen; QIAprep(r) Turbo BioRobot Kit. Human 293 cell culture cells were cultured in 75 ml tissue culture flasks in DMEM and 10% fetal calf serum. At 90-99% confluence, the cells were splitted at 1:3 ratio and plated onto poly-D-lysine (Sigma) coated 96-Well plates. Cells were transfected with 100-500 ng plasmid using lipofectamine 2000 (Invitrogen). After 6 hours, the medium was exchanged for fresh complete growth medium. 24 hours after transfection, the cells were washed twice with DMEM without cysteine and methionine (Invitrogen), supplemented with 1 % dialysed Bovine serum (Sigma) with 50 microgram per ml Heparin (Sigma) and glutamine. The cells were labeled radioactively ('S35 Met-label', from Hartmann Analytic GmbH). After 12 hours, aliquots of the supernatants were harvested in 96-well PCR plates and subjected to SDS gel electrophoresis in precast 420% gradient polyacrylamide Criterion gels (Biorad) under reducing conditions, using Criterion Dodeca Cell gel running chamber (Biorad). The gels were fixed in 10% acetic acid, 25% isopropanol for 30 min, soaked 15-30 min in.AMPLIFY reagent (Amersham), dried and exposed to X-OMAT (AR) film (Kodak). Positive clones were identified and regrown in 96-well-plates. DNA of individual clones was prepared and used for transfection as described above. If one of the clones yielded proteins of the same size as that of the original pool, a positive done was identified. Positive clones were partially sequenced from the 5' end (SEQLAB, Goettingen).

### Example 2: Identification of the human DG153 (comparison) or DG177 homologous nucleic acid and proteins

The term "polynucleotide comprising the nucleotide sequence as shown in GenBank Accession number" relates to the expressible gene of the nucleotide sequences deposited under the corresponding GenBank Accession number. The term "GenBank Accession number" relates to NCBI GenBank database entries (Ref.: Benson et al., Nucleic Acids Res. 28 (2000) 15-18).

DG153 or DG177 homologous proteins and nucleic add molecules coding therefore are obtainable from insect or vertebrate species, e.g. mammals or birds. Particularly preferred are nucleic acids comprising human DG153 or DG177 homologs. The following mouse sequences were identified in the secreted factor screen': Mus musculus arginine-rich, mutated in early stage tumors (Armet), GenBank Accession Number NM_029103 (838 base pairs mRNA) and GenBank Accession Number NP_083379" (179 amino acid protein)), and Mus musculus follistatin-like 1 (Fstl1), GenBank Accession Number NM_008047 and GenBank Accession Number NP_032073.

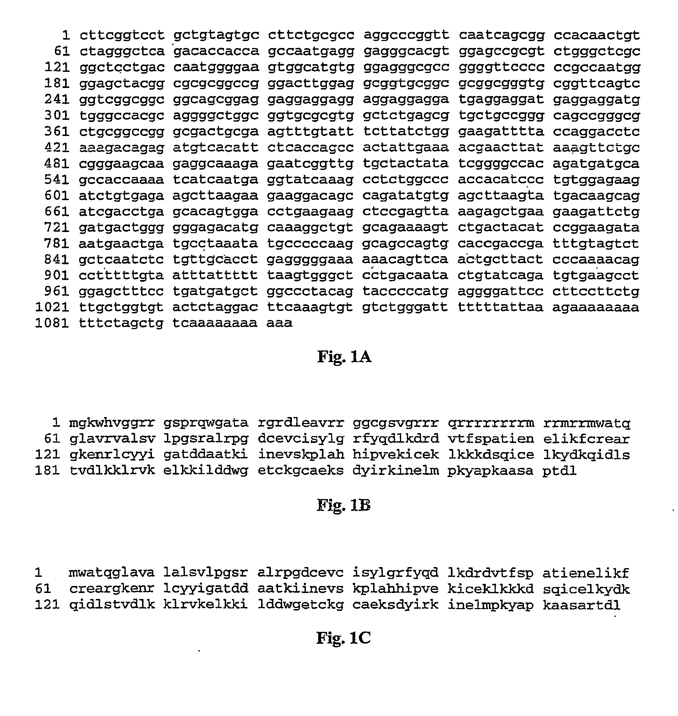

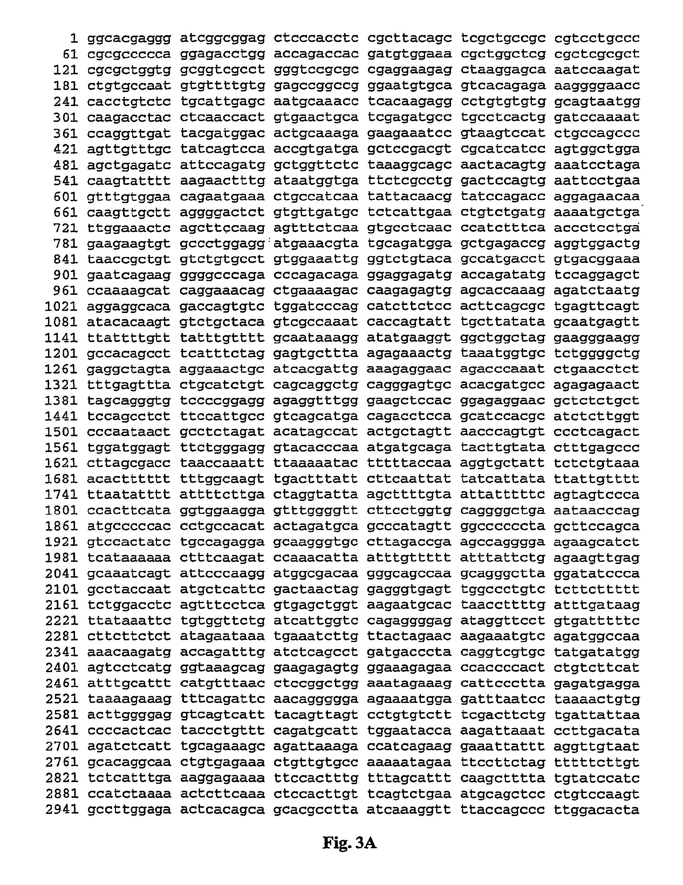

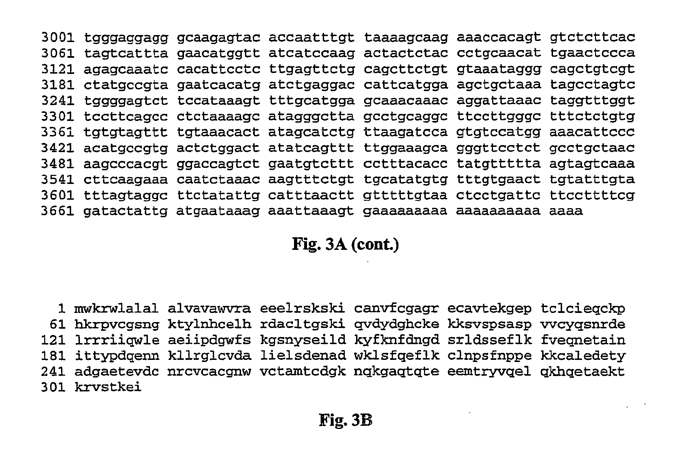

Sequences homologous to mouse DG153 or DG177 were identified using the publicly available program BLASTP 2.2.3 of the non-redundant protein data base of the National Center for Biotechnology Information (NCBI) (see, Altschul et al., 1997, Nucleic Acids Res. 25: 3389-3402). The best human homologs of mouse DG153 are Homo sapiens arginine-rich protein (ARP) gene GenBank Accession Number M83751 (1103 base pairs mRNA; SEQ ID NO: 1; see Fig. 1A), GenBank Accession Number AAB08753.1 (234 amino acid protein; SEQ ID NO: 2; see Fig. 1 B), and Swiss-Prot Accession Number P55145 (179 amino acid protein; SEQ ID NO: 3; see Fig. 1C). The 234 amino acid protein is a longer sequence with arginine-rich regions thought to be the target of cancer-causing mutation. The best human homolog of mouse DG177 is GenBank Accession Number NM_007085 (SEQ ID NO: 4; see Fig. 3A) and GenBank Accession Number NP_009016 (SEQ ID NO: 5; see Fig. 3B).

### Example 3: Expression of the polypeptides in mammalian tissues

To analyse the expression of the polypeptides disclosed herein in mammalian tissues, several mouse strains (preferably mice strains C57BI/6J, C57BI/6 ob/ob and C57BI/KS db/db, and Non-Obese-Diabetic (NOD) mice which are standard model systems in obesity and diabetes research) were purchased from Harian Winkelmann (33178 Borchen, Germany) and Taconic M & B (Germantown, NY 12526, U.S.A.), respectively, and maintained under constant temperature (preferably 22°C), 40 per cent humidity and a light/dark cycle of preferably 14 / 10 hours. The mice were fed a standard chow (for example, from ssniff Spezialitäten GmbH, order number ssniff M-Z V1126-000). For the fasting experiment ("fasted wild-type mice"), wild-type mice were starved for 48 h without food, but only water supplied ad libitum (see, for example, Schnetzler et al., (1993) J Clin Invest 92: 272-280, Mizuno et al., (1996) Proc Natl Acad Sci 93: 3434-3438). In a further experiment wild-type (wt) mice were fed a control diet (preferably Altromin C1057 mod control, 4.5% crude fat) or high fat diet (preferably Altromin C1057mod. high fat, 23.5% crude fat). Animals were sacrificed at an age of 6 to 8 weeks. The animal tissues were isolated according to standard procedures known to those skilled in the art, snap frozen in liquid nitrogen and stored at -80°C until needed.

For analyzing the role of the proteins disclosed herein in the in vitro differentiation of different mammalian cell culture cells for the conversion of pre-adipocytes to adipocytes, mammalian fibroblast (3T3-L1) cells (e.g., Green & Kehinde, Cell 1: 113-116, 1974) were obtained from the American Tissue Culture Collection (ATCC, Hanassas, VA, USA; ATCC- CL 173). 3T3-L1 cells were maintained as fibroblasts and differentiated into adipocytes as described in the prior art (e.g., Qiu. et al., J. Biol. Chem. 276: 11988-11995, 2001; Slieker et al., BBRC 251: 225-229, 1998). In brief, cells were plated in DMEM/10% FCS (Invitrogen, Karlsruhe, Germany) at 50,000 cells/well in duplicates in 6-well plastic dishes and culture in a humidified atmosphere of 5% CO₂ at 37°C. At confluence (defined as day 0: d0) cells were transferred to serum-free (SF) medium, containing DMEM/HamF12 (3:1; Invitrogen), fetuin (300 µg/ml; Sigma, Munich, Germany), transferrin (2 µg/ml; Sigma), pantothenate (17 µM; Sigma), biotin (1 µM; Sigma), and EGF (0.8nM; Hoffmann-La Roche, Basel, Switzerland). Differentiation was induced by adding dexamethasone (DEX; 1. µM; Sigma), 3-methyl-isobutyl-1-methylxanthine (MIX; 0.5mM; Sigma), and bovine insulin (5 µg/ml; Invitrogen). Four days after confluence (d4), cells were kept in SF medium, containing bovine insulin (5 µg/ml) until differentiation was completed. At various time points of the differentiation procedure, beginning with day 0 (day of confluence) and day 2 (hormone addition; for example, dexamethasone and 3-isobutyl-1-methylxanthine), up to 10 days of differentiation, suitable aliquots of cells were taken every two days.

RNA was isolated from mouse tissues or cell culture cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferably using Superscript II RNaseH- Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

The following prime/probe pairs were used for the TaqMan analysis (GenBank Accession Number NM_029103 for the mouse DG153 sequence): Mouse DG153 forward primer (Seq ID NO:6): 5'- AGA GAA TCG GTT GTG CTA CTA CAT TG -3'; mouse DG153 reverse primer (Seq ID NO:7): 5'- GGC TTC GAC ACC TCA TTG ATG -3'; mouse DG153 Taqman probe (Seq ID NO:8): (5/6-FAM) AGC CAC AGA TGA TGC TGC CAC CAA (5/6-TAMRA).

The following prime/probe pairs were used for the TaqMan analysis (GenBank Accession Number NM_008047 for the mouse DG177 sequence): Mouse DG177 forward primer (Seq ID NO:9): 5'- GAA GTC TGC GAG TCC ATC TGC -3'; mouse DG177 reverse primer (Seq ID NO:10): 5'- GCG CCG TCG GAG CTC -3'; mouse DG177 Taqman probe (Seq ID NO:11): (5/6-FAM)-AGC CCA GTT GTC TGC TAT CAA GCT AAC CG- (5/6-TAMRA).

The function of the mammalian DG153 and DG177 in metabolism was further validated by analyzing the expression of the transcripts in different tissues and by analyzing the role in adipocyte differentiation.

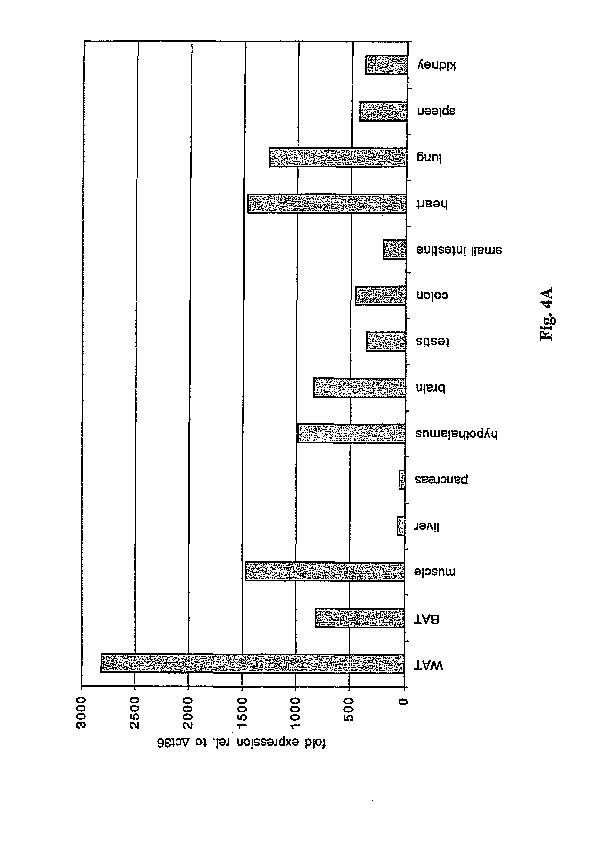

Expression profiling studies confirm the particular relevance of DG177 as regulators of energy metabolism in mammals. DG177 is expressed in several mammalian tissues, with highest expression levels in white adipose tissue (WAT) in wild type mice. In addition, DG177 is also highly expressed in muscle, heart, lung, and at lower, but robust levels in brown adipose tissue (BAT), hypothalamus and brain in wild type mice, as depicted in Fig. 4A.

We used mouse models of insulin resistance and/or diabetes, such as mice carrying gene knockouts in the leptin pathway (for example, ob/ob (leptin) or db (leptin receptor/ligand) mice) to study the expression of DG177. Such mice develop typical symptoms of diabetes, show hepatic lipid accumulation and frequently have increased plasma lipid levels (see Bruning et al, 1998, Mol. Cell. 2: 559-569). We found, that the expression of DG177 is significantly up-regulated in metabolic active tissue (BAT) in genetically induced obese mice (ob/ob) compared to wild type mice (see Fig. 4B). We also found, that the expression of DG177 is down-regulated in WAT and liver of fasted wild type mice, compared to wild type mice fed a standard diet (Fig. 4B).

Expression of DG177 mRNA was also examined in susceptible wild type mice (for example, C57BI/6) that show symptoms of diabetes, lipid accumulation, and high plasma lipid levels, if fed a high fat diet. The expression of DG177 is significantly up-regulated in WAT and muscle (see Fig. 4C) supporting that DG177 is involved in the regulation of mammalian metabolism.

In non-obese diabetic (NOD) mice, expression of DG177 is significantly down-regulated in white adipose tissue compared to wild type mice, as shown in Fig. 4D.

We show in this invention (see Fig. 4E) that the DG177 mRNA is expressed and down-regulated during the differentiation of preadipocytes into mature adipocyctes. Therefore, the DG177 protein might also play an essential role in adipogenesis.

## Claims

1. A pharmaceutical composition comprising a protein as shown in SEQ ID NO:5 and/or a nucleic acid molecule wherein said nucleic acid molecule is selected from the group consisting of
(a) a nucleic acid molecule encoding a polypeptide as shown in SEQ ID NO: 5;
(b) a nucleic acid molecule which comprises or is the nucleic acid molecule as shown in SEQ ID NO: 4;
(c) a nucleic acid molecule being degenerate with as a result of the genetic code to the nucleic acid sequences as defined in (a) or (b),
(d) a nucleic acid molecule that hybridizes at 50°C in a solution containing 1 x SSC and 0.1% SDS to a nucleic acid molecule as defined in (a) to (c) and/or a nucleic acid molecule which is complementary thereto;
(e) a nucleic acid molecule that encodes a polypeptide which is at least 85%, preferably at least 90%, more preferably at least 95%, more preferably at least 98% and up to 99,6% identical to the human DG177, to a polypeptide as defined in (a), wherein the composition optionally contains pharmaceutically acceptable carriers, diluents, and/or additives, for use in the treatment of pancreatic diseases selected from the group consisting of diabetes, such as insulin-dependent diabetes mellitus or non-insulin-dependent diabetes mellitus, obesity and/or metabolic syndrome.

2. The composition for use according to claim 1, wherein said nucleic acid encodes a polypeptide contributing to regulating the metabolism, in particular human metabolism.

3. The composition for use according to any one of claims 1-2 for the modulation of pancreatic development, in particular for the regeneration of pancreatic tissues or cells, particularly pancreatic beta cells.

4. A method of identifying a (poly)peptide involved in the regulation of energy homeostasis and/or metabolism in a mammal, comprising the steps of
(a) contacting a collection of (poly)peptides with a polypeptide as shown in SEQ ID NO:5 under conditions that allow binding of said (poly)peptides;
(b) removing (poly)peptides which do not bind and
(c) identifying (poly)peptides that bind to said polypeptide as shown in SEQ ID NO:5.

5. A method for screening for an agent, which effects/modulates the interaction of a polypeptide as shown in SEQ ID NO:5 with a binding target comprising the steps of
(a) incubating a mixture comprising
(aa) a polypeptide as shown in SEQ ID NO:5;
(ab) a binding target/agent of said polypeptide as shown in SEQ ID NO:5; and
(ac) a candidate agent under conditions whereby said polypeptide specifically binds to said binding target at a reference affinity;
(b) detecting the binding affinity of said polypeptide as shown in SEQ ID NO:5 to said binding target to determine an affinity for the agent; and
(c) determining a difference between affinity for the agent and reference affinity.

6. A method for screening for an agent, which effects/modulates the activity of a polypeptide as shown in SEQ ID NO:5, comprising the steps of
(a) incubating a mixture comprising
(aa) a polypeptide as shown in SEQ ID NO:5;
(ab) a candidate agent under conditions whereby said polypeptide as shown in SEQ ID NO:5 exhibits a reference activity,
(b) detecting the activity of said polypeptide as shown in SEQ ID NO:5 or functional fragment thereof to determine an activity for the agent; and
(c) determining a difference between activity for the agent and reference activity.

## Patentansprüche

1. Pharmazeutische Zusammensetzung, umfassend ein Protein wie in SEQ ID NO:5 gezeigt und/oder ein Nukleinsäuremolekül, wobei das Nukleinsäuremolekül ausgewählt ist aus der Gruppe, bestehend aus,
(a) einem Nukleinsäuremolekül, welches ein Polypeptid wie in SEQ ID NO:5 gezeigt kodiert;
(b) einem Nukleinsäuremolekül, welches das in SEQ ID NO:4 gezeigte Nukleinsäuremolekül umfasst oder dieses ist;
(c) einem Nukleinsäuremolekül, welches als Ergebnis des genetischen Kodes zu den in (a) und (b) definierten Nukleinsäuresequenzen degeneriert ist;
(d) einem Nukleinsäuremolekül, welches bei 50°C in einer Lösung, die 1 x SSC und 0,1% SDS enthält, an ein in (a) bis (c) definiertes Nukleinsäuremolekül und/oder an ein dazu komplementäres Nukleinsäuremolekül hybridisiert;
(e) einem Nukleinsäuremolekül, das ein Polypeptid kodiert, das zu mindestens 85%, bevorzugt zu mindestens 90%, weiter bevorzugt zu mindestens 95%, weiter bevorzugt zu mindestens 98% und bis zu 99,6% identisch ist zu dem humanen DG177, zu einem wie in (a) definierten Polypeptid, wobei die Zusammensetzung wahlweise pharmazeutisch annehmbare Träger, Verdünner und/oder Zusätze enthält, zur Verwendung bei der Behandlung von Pankreaserkrankungen, ausgewählt aus der Gruppe bestehend aus Diabetes wie etwa Insulin-abhängiger Diabetes mellitus und/oder nicht-Insulin-abhängiger Diabetes mellitus, Obesitas und/oder metabolisches Syndrom.

2. Zusammensetzung zur Verwendung nach Anspruch 1, wobei die Nukleinsäure ein Polypeptid, welches zum Regulieren des Metabolismus, im Besonderen menschlichen Metabolismus, beiträgt, kodiert.

3. Zusammensetzung zur Verwendung nach einem der Ansprüche 1-2 für die Modulierung von pankreatischer Entwicklung, im Besonderen für die Regenerierung von Pankreasgewebe oder -zellen, insbesondere pankreatischen beta-Zellen.

4. Verfahren des Identifizierens eines (Poly)peptids, welches in der Regulation der Energiehomöostase und/oder Metabolismus in einem Säugetier involviert ist, umfassend die Schritte von
(a) Kontaktieren einer Sammlung von (Poly)peptiden mit einem Polypeptid wie in SEQ ID NO:5 gezeigt unter Bedingungen, die Binden des (Poly)peptids erlauben;
(b) Entfernen der (Poly)peptide, die nicht binden, und
(c) Identifizieren von (Poly)peptiden, die an das Polypeptid wie in SEQ ID NO:5 gezeigt binden.

5. Verfahren zum Screening nach einem Wirkstoff, welcher die Interaktion eines Polypeptids wie in SEQ ID NO: 5 gezeigt mit einem Bindungs-Zielobjekt bewirkt/moduliert, umfassend die Schritte von:
(a) Inkubieren eines Gemischs, umfassend
(aa) ein Polypeptid wie in SEQ ID NO: 5 gezeigt;
(ab) ein/en Bindungs-Zielobjekt/-wirkstoff des Polypeptids wie in SEQ ID NO: 5 gezeigt; und
(ac) einen Wirkstoffkandidaten unter Bedingungen, bei denen das Polypeptid spezifisch an das Bindungs-Zielobjekt mit einer Referenzaffinität bindet,
(b) Detektieren der Bindungsaffinität des Polypeptids wie in SEQ ID NO: 5 gezeigt an das Bindungszielobjekt, um eine Affinität für den Wirkstoff zu bestimmen; und
(c) Bestimmen eines Unterschiedes zwischen Affinität für den Wirkstoff und Referenzaffinität.

6. Verfahren zum Screening nach einem Wirkstoff, welcher die Aktivität eines Polypeptids wie in SEQ ID NO: 5 gezeigt bewirkt/moduliert, umfassend die Schritte:
(a) Inkubieren eines Gemischs umfassend
(aa) ein Polypeptid wie in SEQ ID NO: 5 gezeigt;
(ab) einen Wirkstoffkandidaten unter Bedingungen, bei denen das Polypeptid wie in SEQ ID NO: 5 gezeigt eine Referenzaktivität aufweist,
(b) Detektieren der Aktivität des Polypeptids wie in SEQ ID NO: 5 gezeigt oder funktionellen Fragments davon, um eine Aktivität für den Wirkstoff zu bestimmen; und
(c) Bestimmen eines Unterschiedes zwischen Aktivität für den Wirkstoff und Referenzaktivität.

## Revendications

1. Composition pharmaceutique comprenant une protéine comme montrée dans la SEQ ID NO:5 et/ou une molécule d'acide nucléique, ladite molécule d'acide nucléique étant sélectionnée dans le groupe constitué de
(a) une molécule d'acide nucléique codant un polypeptide comme montrée dans la SEQ ID NO:5 ;
(b) une molécule d'acide nucléique qui comprend ou est la molécule d'acide nucléique comme montrée dans la SEQ ID NO:4 ;
(c) une molécule d'acide nucléique dégénérée suite au code génétique en les séquences d'acide nucléique comme définies en (a) ou (b) ;
(d) une molécule d'acide nucléique qui hybride à 50°C dans une solution contenant 1 x SSC et 0,1% de SDS avec une molécule d'acide nucléique comme définie en (a) à (c) et/ou une molécule d'acide nucléique qui est complémentaire de celle-ci ;
(e) une molécule d'acide nucléique qui code un polypeptide qui est au moins identique à 85%, de préférence au moins à 90%, davantage préféré au moins à 95%, encore davantage préféré au moins à 98% et jusqu'à 99,6% à la DG177 humaine, à un polypeptide comme défini en (a),
la composition contenant facultativement des supports, diluants et/ou additifs pharmaceutiquement acceptables, pour l'utilisation dans le traitement de maladies du pancréas sélectionnées dans le groupe constitué du diabète, comme le diabète sucré insulino-dépendant et/ou le diabète sucré non insulino-dépendant, l'obésité et/ou le syndrome métabolique.

2. Composition pour l'utilisation selon la revendication 1, dans laquelle ledit acide nucléique code un polypeptide contribuant à la régulation du métabolisme, en particulier du métabolisme humain.

3. Composition pour l'utilisation selon l'une quelconque des revendications 1-2 pour la modulation du développement du pancréas, en particulier pour la régénération des tissus ou des cellules du pancréas, en particulier les cellules bêta du pancréas.

4. Procédé d'identification d'un (poly)peptide impliqué dans la régulation de l'homéostasie et/ou du métabolisme énergétique chez un mammifère, comprenant les étapes de
(a) mise en contact d'une collection de (poly)peptides avec un polypeptide comme montré dans la SEQ ID NO:5 sous des conditions qui permettent la liaison desdits (poly)peptides ;
(b) l'élimination desdits (poly)peptides qui ne se lient pas ; et
(c) l'identification des (poly)peptides qui se lient audit polypeptide comme montré dans la SEQ ID NO:5.

5. Procédé de criblage d'un agent, qui affecte/module l'interaction d'un polypeptide comme montré dans la SEQ ID NO:5 avec une cible de liaison, comprenant les étapes de :
(a) incubation d'un mélange comprenant
(aa) un polypeptide comme montré dans la SEQ ID NO:5 ;
(ab) un agent/cible de liaison dudit polypeptide comme montré dans SEQ ID NO:5 ; et
(ac) un agent candidat sous des conditions dans lesquelles ledit polypeptide se lie spécifiquement à ladite cible de liaison à une affinité de référence ;
(b) détection de l'affinité de liaison dudit polypeptide comme montré dans SEQ ID NO:5 à ladite cible de liaison pour déterminer une affinité pour l'agent ; et
(c) détermination d'une différence entre l'affinité pour l'agent et l'affinité de référence.

6. Procédé de criblage d'un agent, qui affecte/module l'activité d'un polypeptide comme montré dans la SEQ ID NO:5, comprenant les étapes de
(a) incubation d'un mélange comprenant (aa) un polypeptide comme montré dans la SEQ ID NO:5 ; (ab) un agent candidat sous des conditions dans lesquelles ledit polypeptide comme montré dans la SEQ ID NO:5 montre une activité de référence ;
(b) détection de l'activité dudit polypeptide comme montré dans la SEQ ID NO:5 ou d'un fragment fonctionnel de celui-ci pour déterminer une activité pour l'agent ; et
(c) détermination d'une différence entre l'activité pour l'agent et l'activité de référence.


### SEQUENCE LISTING

<110> Develogen Aktiengesellschaft fur entwicklungsbiolo
<120> Use of DG153 or DG177 secreted protein products for preventing and treating pancreatic diseases and/or obesity and/or metabolic syndrome
<130> 31130PWO WWHC
<140> PCT/EP2004/007531
   <141> 2004-07-08
<150> EP03015883.6
   <151> 2003-07-11
<150> EP03016710.0
   <151> 2003-07-22
<160> 11
<170> PatentIn Ver. 2.1
<210> 1
   <211> 1103
   <212> DNA
   <213> human
<220>
   <223> nucleotide sequence of human DG153 protein
<400> 1
   cttcggtcct gctgtagtgc cttctgcgcc aggcccggtt caatcagcgg ccacaactgt 60
   ctagggctca gacaccacca gccaatgagg gagggcacgt ggagccgcgt ctgggctcgc 120
   ggctcctgac caatggggaa gtggcatgtg ggagggcgcc ggggttcccc ccgccaatgg 180
   ggagctacgg cgcgcggccg ggacttggag gcggtgcggc gcggcgggtg cggttcagtc 240
   ggtcggcggc ggcagcggag gaggaggagg aggaggagga tgaggaggat gaggaggatg 300
   tgggccacgc aggggctggc ggtgcgcgtg gctctgagcg tgctgccggg cagccgggcg 360
   ctgcggccgg gcgactgcga agtttgtatt tcttatctgg gaagatttta ccaggacctc 420
   aaagacagag atgtcacatt ctcaccagcc actattgaaa acgaacttat aaagttctgc 480
   cgggaagcaa gaggcaaaga gaatcggttg tgctactata tcggggccac agatgatgca 540
   gccaccaaaa tcatcaatga ggtatcaaag cctctggccc accacatccc tgtggagaag 600
   atctgtgaga agcttaagaa gaaggacagc cagatatgtg agcttaagta tgacaagcag 660
   atcgacctga gcacagtgga cctgaagaag ctccgagtta aagagctgaa gaagattctg 720
   gatgactggg gggagacatg caaaggctgt gcagaaaagt ctgactacat ccggaagata 780
   aatgaactga tgcctaaata tgcccccaag gcagccagtg caccgaccga tttgtagtct 840
   gctcaatctc tgttgcacct gagggggaaa aaacagttca actgcttact cccaaaacag 900
   cctttttgta atttattttt taagtgggct cctgacaata ctgtatcaga tgtgaagcct 960
   ggagctttcc tgatgatgct ggccctacag tacccccatg aggggattcc cttccttctg 1020
   ttgctggtgt actctaggac ttcaaagtgt gtctgggatt tttttattaa agaaaaaaaa 1080
   tttctagctg tcaaaaaaaa aaa                                         1103
<210> 2
   <211> 234
   <212> PRT
   <213> human
<220>
   <223> amino acid sequence of human DG153 protein, longer variant
<400> 2
   Met Gly Lys Trp His Val Gly Gly Arg Arg Gly Ser Pro Arg Gln Trp
    1               5                   10                  15
   Gly Ala Thr Ala Arg Gly Arg Asp Leu Glu Ala Val Arg Arg Gly Gly
               20                  25                  30
   Cys Gly Ser Val Gly Arg Arg Arg Gln Arg Arg Arg Arg Arg Arg Arg
           35                  40                  45
   Arg Met Arg Arg Met Arg Arg Met Trp Ala Thr Gln Gly Leu Ala Val
       50                  55                  60
   Arg Val Ala Leu Ser Val Leu Pro Gly Ser Arg Ala Leu Arg Pro Gly
    65                  70                  75                  80
   Asp Cys Glu Val Cys Ile Ser Tyr Leu Gly Arg Phe Tyr Gln Asp Leu
                   85                  90                  95
   Lys Asp Arg Asp Val Thr Phe Ser Pro Ala Thr Ile Glu Asn Glu Leu
               100                 105                 110
   Ile Lys Phe Cys Arg Glu Ala Arg Gly Lys Glu Asn Arg Leu Cys Tyr
           115                 120                 125
   Tyr Ile Gly Ala Thr Asp Asp Ala Ala Thr Lys Ile Ile Asn Glu Val
       130                 135                 140
   Ser Lys Pro Leu Ala His His Ile Pro Val Glu Lys Ile Cys Glu Lys
   145                 150                 155                 160
   Leu Lys Lys Lys Asp Ser Gln Ile Cys Glu Leu Lys Tyr Asp Lys Gln
                   165                 170                 175
   Ile Asp Leu Ser Thr Val Asp Leu Lys Lys Leu Arg Val Lys Glu Leu
               180                 185                 190
   Lys Lys Ile Leu Asp Asp Trp Gly Glu Thr Cys Lys Gly Cys Ala Glu
           195                 200                 205
   Lys Ser Asp Tyr Ile Arg Lys Ile Asn Glu Leu Met Pro Lys Tyr Ala
       210                 215                 220
   Pro Lys Ala Ala Ser Ala Pro Thr Asp Leu
   225                 230
<210> 3
   <211> 179
   <212> PRT
   <213> human
<220>
   <223> amino acid sequence of human DG153 protein, shorter variant
<400> 3
<210> 4
   <211> 3714
   <212> DNA
   <213> human
<220>
   <223> nucleotide sequence of human DG177 protein
<400> 4
   ggcacgaggg atcggcggag ctcccacctc cgcttacagc tcgctgccgc cgtcctgccc 60
   cgcgccccca ggagacctgg accagaccac gatgtggaaa cgctggctcg cgctcgcgct 120
   cgcgctggtg gcggtcgcct gggtccgcgc cgaggaagag ctaaggagca aatccaagat 180
   ctgtgccaat gtgttttgtg gagccggccg ggaatgtgca gtcacagaga aaggggaacc 240
   cacctgtctc tgcattgagc aatgcaaacc tcacaagagg cctgtgtgtg gcagtaatgg 300
   caagacctac ctcaaccact gtgaactgca tcgagatgcc tgcctcactg gatccaaaat 360
   ccaggttgat tacgatggac actgcaaaga gaagaaatcc gtaagtccat ctgccagccc 420
   agttgtttgc tatcagtcca accgtgatga gctccgacgt cgcatcatcc agtggctgga 480
   agctgagatc attccagatg gctggttctc taaaggcagc aactacagtg aaatcctaga 540
   caagtatttt aagaactttg ataatggtga ttctcgcctg gactccagtg aattcctgaa 600
   gtttgtggaa cagaatgaaa ctgccatcaa tattacaacg tatccagacc aggagaacaa 660
   caagttgctt aggggactct gtgttgatgc tctcattgaa ctgtctgatg aaaatgctga 720
   ttggaaactc agcttccaag agtttctcaa gtgcctcaac ccatctttca accctcctga 780
   gaagaagtgt gccctggagg atgaaacgta tgcagatgga gctgagaccg aggtggactg 840
   taaccgctgt gtctgtgcct gtggaaattg ggtctgtaca gccatgacct gtgacggaaa 900
   gaatcagaag ggggcccaga cccagacaga ggaggagatg accagatatg tccaggagct 960
   ccaaaagcat caggaaacag ctgaaaagac caagagagtg agcaccaaag agatctaatg 1020
   aggaggcaca gaccagtgtc tggatcccag catcttctcc acttcagcgc tgagttcagt 1080
   atacacaagt gtctgctaca gtcgccaaat caccagtatt tgcttatata gcaatgagtt 1140
   ttattttgtt tatttgtttt gcaataaagg atatgaaggt ggctggctag gaagggaagg 1200
   gccacagcct tcatttctag gagtgcttta agagaaactg taaatggtgc tctggggctg 1260
   gaggctagta aggaaactgc atcacgattg aaagaggaac agacccaaat ctgaacctct 1320
   tttgagttta ctgcatctgt cagcaggctg cagggagtgc acacgatgcc agagagaact 1380
   tagcagggtg tccccggagg agaggtttgg gaagctccac ggagaggaac gctctctgct 1440
   tccagcctct ttccattgcc gtcagcatga cagacctcca gcatccacgc atctcttggt 1500
   cccaataact gcctctagat acatagccat actgctagtt aacccagtgt ccctcagact 1560
   tggatggagt ttctgggagg gtacacccaa atgatgcaga tacttgtata ctttgagccc 1620
   cttagcgacc taaccaaatt ttaaaaatac tttttaccaa aggtgctatt tctctgtaaa 1680
   acactttttt tttggcaagt tgactttatt cttcaattat tatcattata ttattgtttt 1740
   ttaatatttt attttcttga ctaggtatta agcttttgta attatttttc agtagtccca 1800
   ccacttcata ggtggaagga gtttggggtt cttcctggtg caggggctga aataacccag 1860
   atgcccccac cctgccacat actagatgca gcccatagtt ggccccccta gcttccagca 1920
   gtccactatc tgccagagga gcaagggtgc cttagaccga agccagggga agaagcatct 1980
   tcataaaaaa ctttcaagat ccaaacatta atttgttttt atttattctg agaagttgag 2040
   gcaaatcagt attcccaagg atggcgacaa gggcagccaa gcagggctta ggatatccca 2100
   gcctaccaat atgctcattc gactaactag gagggtgagt tggccctgtc tcttcttttt 2160
   tctggacctc agtttcctca gtgagctggt aagaatgcac taaccttttg atttgataag 2220
   ttataaattc tgtggttctg atcattggtc cagaggggag ataggttcct gtgatttttc 2280
   cttcttctct atagaataaa tgaaatcttg ttactagaac aagaaatgtc agatggccaa 2340
   aaacaagatg accagatttg atctcagcct gatgacccta caggtcgtgc tatgatatgg 2400
   agtcctcatg ggtaaagcag gaagagagtg ggaaagagaa ccaccccact ctgtcttcat 2460
   atttgcattt catgtttaac ctccggctgg aaatagaaag cattccctta gagatgagga 2520
   taaaagaaag tttcagattc aacaggggga agaaaatgga gatttaatcc taaaactgtg 2580
   acttggggag gtcagtcatt tacagttagt cctgtgtctt tcgacttctg tgattattaa 2640
   ccccactcac taccctgttt cagatgcatt tggaatacca aagattaaat ccttgacata 2700
   agatctcatt tgcagaaagc agattaaaga ccatcagaag gaaattattt aggttgtaat 2760
   gcacaggcaa ctgtgagaaa ctgttgtgcc aaaaatagaa ttccttctag tttttcttgt 2820
   tctcatttga aaggagaaaa ttccactttg tttagcattt caagctttta tgtatccatc 2880
   ccatctaaaa actcttcaaa ctccacttgt tcagtctgaa atgcagctcc ctgtccaagt 2940
   gccttggaga actcacagca gcacgcctta atcaaaggtt ttaccagccc ttggacacta 3000
   tgggaggagg gcaagagtac accaatttgt taaaagcaag aaaccacagt gtctcttcac 3060
   tagtcattta gaacatggtt atcatccaag actactctac cctgcaacat tgaactccca 3120
   agagcaaatc cacattcctc ttgagttctg cagcttctgt gtaaataggg cagctgtcgt 3180
   ctatgccgta gaatcacatg atctgaggac cattcatgga agctgctaaa tagcctagtc 3240
   tggggagtct tccataaagt tttgcatgga gcaaacaaac aggattaaac taggtttggt 3300
   tccttcagcc ctctaaaagc atagggctta gcctgcaggc ttccttgggc tttctctgtg 3360
   tgtgtagttt tgtaaacact atagcatctg ttaagatcca gtgtccatgg aaacattccc 3420
   acatgccgtg actctggact atatcagttt ttggaaagca gggttcctct gcctgctaac 3480
   aagcccacgt ggaccagtct gaatgtcttt cctttacacc tatgttttta agtagtcaaa 3540
   cttcaagaaa caatctaaac aagtttctgt tgcatatgtg tttgtgaact tgtatttgta 3600
   tttagtaggc ttctatattg catttaactt gtttttgtaa ctcctgattc ttccttttcg 3660
   gatactattg atgaataaag aaattaaagt gaaaaaaaaa aaaaaaaaaa aaaa       3714
<210> 5
   <211> 308
   <212> PRT
   <213> human
<220>
   <223> amino acid sequence of human DG177 protein
<400> 5
   Met Trp Lys Arg Trp Leu Ala Leu Ala Leu Ala Leu Val Ala Val Ala
    1               5                  10                  15
   Trp Val Arg Ala Glu Glu Glu Leu Arg Ser Lys Ser Lys Ile Cys Ala
               20                  25                  30
   Asn Val Phe Cys Gly Ala Gly Arg Glu Cys Ala Val Thr Glu Lys Gly
           35                  40                  45
   Glu Pro Thr Cys Leu Cys Ile Glu Gln Cys Lys Pro His Lys Arg Pro
       50                  55                  60
   Val Cys Gly Ser Asn Gly Lys Thr Tyr Leu Asn His Cys Glu Leu His
   65                  70                  75                  80
   Arg Asp Ala Cys Leu Thr Gly Ser Lys Ile Gln Val Asp Tyr Asp Gly
                   85                  90                  95
   His Cys Lys Glu Lys Lys Ser Val Ser Pro Ser Ala Ser Pro Val Val
               100                 105                 110
   Cys Tyr Gln Ser Asn Arg Asp Glu Leu Arg Arg Arg Ile Ile Gln Trp
           115                 120                 125
   Leu Glu Ala Glu Ile Ile Pro Asp Gly Trp Phe Ser Lys Gly Ser Asn
       130                 135                 140
   Tyr Ser Glu Ile Leu Asp Lys Tyr Phe Lys Asn Phe Asp Asn Gly Asp
   145                 150                 155                 160
   Ser Arg Leu Asp Ser Ser Glu Phe Leu Lys Phe Val Glu Gln Asn Glu
                   165                 170                 175
   Thr Ala Ile Asn Ile Thr Thr Tyr Pro Asp Gln Glu Asn Asn Lys Leu
               180                 185                 190
   Leu Arg Gly Leu Cys Val Asp Ala Leu Ile Glu Leu Ser Asp Glu Asn
           195                 200                 205
   Ala Asp Trp Lys Leu Ser Phe Gln Glu Phe Leu Lys Cys Leu Asn Pro
       210                 215                 220
   Ser Phe Asn Pro Pro Glu Lys Lys Cys Ala Leu Glu Asp Glu Thr Tyr
   225                 230                 235                 240
   Ala Asp Gly Ala Glu Thr Glu Val Asp Cys Asn Arg Cys Val Cys Ala
                   245                 250                 255
   Cys Gly Asn Trp Val Cys Thr Ala Met Thr Cys Asp Gly Lys Asn Gln
               260                 265                 270
   Lys Gly Ala Gln Thr Gln Thr Glu Glu Glu Met Thr Arg Tyr Val Gln
           275                 280                 285
   Glu Leu Gln Lys His Gln Glu Thr Ala Glu Lys Thr Lys Arg Val Ser
       290                 295                 300
   Thr Lys Glu Ile
   305
<210> 6
   <211> 26
   <212> DNA
   <213> Artificial Sequence
<220>
   <223> Description of Artificial Sequence: primer
<220>
   <223> mouse DG153 forward primer
<400> 6
   agagaatcgg ttgtgctact acattg 26
<210> 7
   <211> 21
   <212> DNA
   <213> Artificial Sequence
<220>
   <223> Description of Artificial Sequence: primer
<220>
   <223> mouse DG153 reverse primer
<400> 7
   ggcttcgaca cctcattgat g 21
<210> 8
   <211> 24
   <212> DNA
   <213> Artificial Sequence
<220>
   <223> Description of Artificial Sequence: primer
<220>
   <223> mouse DG153 Taqman probe
<400> 8
   agccacagat gatgctgcca ccaa 24
<210> 9
   <211> 21
   <212> DNA
   <213> Artificial Sequence
<220>
   <223> Description of Artificial Sequence: primer
<220>
   <223> mouse DG177 forward primer
<400> 9
   gaagtctgcg agtccatctg c 21
<210> 10
   <211> 15
   <212> DNA
   <213> Artificial Sequence
<220>
   <223> Description of Artificial Sequence: primer
<220>
   <223> mouse DG177 reverse primer
<400> 10
   gcgccgtcgg agctc 15
<210> 11
   <211> 29
   <212> DNA
   <213> Artificial Sequence
<220>
   <223> Description of Artificial Sequence: primer
<220>
   <223> mouse DG177 Taqman probe
<400> 11
   agcccagttg tctgctatca agctaaccg 29